# EUROPEAN PATENT SPECIFICATION

(11) **EP 3 438 948 B1**
(45) Date of publication and mention of the grant of the patent: **30.08.2023**
(21) Application number: 17795766.9
(22) Date of filing: 31.01.2017
(51) Int. Cl.: G06V 10/147, G06V 10/20, G06V 40/19, H04N 23/611, B60W 50/14, B60K 35/00, B60W 40/08, G08G 1/04, G06T 7/20, G08G 1/16, G06V 20/59, A61B 3/113

(54) **IMAGE PROCESSING APPARATUS, IMAGE PROCESSING METHOD, AND MOBILE BODY**
BILDVERARBEITUNGSVORRICHTUNG, BILDVERARBEITUNGSVERFAHREN UND MOBILER KÖRPER
APPAREIL DE TRAITEMENT D'IMAGE, PROCÉDÉ DE TRAITEMENT D'IMAGE ET CORPS MOBILE

(30) Priority: 11.05.2016 JP 2016095674
(43) Date of publication of application: 06.02.2019
(73) Proprietor: Sony Group Corporation, Tokyo 108-0075 (JP)
(72) Inventor: OBA, Eiji, Tokyo 108-0075 (JP)
(74) Representative: Witte, Weller & Partner Patentanwälte mbB
(86) International application number: PCT/JP2017/003315
(87) International publication number: WO 2017/195405

(56) References cited:
- EP-A2- 2 979 914
- WO-A1-2005/093679
- WO-A1-2015/174963
- WO-A2-2014/006909
- JP-A- 2003 015 816
- JP-A- 2005 323 180
- JP-A- 2006 043 429
- JP-A- 2006 293 909
- JP-A- 2008 210 239
- JP-A- 2010 142 410
- JP-A- 2010 198 313
- JP-A- 2013 218 671
- JP-A- 2014 179 892
- JP-A- 2014 179 892
- JP-A- 2015 011 579
- JP-A- 2015 116 376
- JP-A- 2016 034 782
- JP-A- 2016 045 707
- JP-A- 2016 048 885
- JP-A- 2016 115 023
- JP-A- 2016 115 356
- JP-A- 2017 023 519
- US-A1- 2015 173 665
- US-A1- 2015 328 985

## Description

### Technical Field

The technology disclosed in the present specification relates to an image processing device and an image processing method that process an image of a driver picked up by an in-vehicle camera.

### Background Art

Systems that grasp the states of drivers using in-vehicle cameras have been known. For example, systems that capture the states of drivers such as the attitudes of heads and blinking with cameras, recognize the states of the drivers to detect dozing or fatigue as early as possible, and urge the drivers to have a rest or the like to prevent unreasonable driving have been discussed, and some of the systems have been manufactured (see, for example, Patent Literatures 1 and 2).

In addition, safety precaution devices or the like that continuously analyze the transition of the operations of accelerator pedals, brake pedals, and steering by drivers in a time-series fashion to observe changes in the movement stabilities, and that estimate and determine the driving attention degrees of the drivers have been examined.

In the future, it is assumed that if advanced driving assistance systems (ADAS) will become further widespread, a ratio at which vehicles are capable of maintaining their traveling without the intervention of drivers increases with a proviso that certain conditions are met during the traveling, a ratio at which the vehicles that autonomously travel will gradually increase, and the drivers are not required to operate steering at all during the traveling of the vehicles. In addition, if traveling dedicated lanes or lane sections in which the intervention of drivers becomes unnecessary will be introduced as social infrastructures, the drivers of vehicles traveling in the sections are consciously separated from a steering loop for forward attention driving, the sequence of the surrounding recognition of the traveling vehicles is delayed, and a traveling time without the recognition increases. That is, if partially or fully automated driving will be introduced, the drivers are not required to turn their visual lines to a forward side with a high attention degree at all times. Consequently, the drivers become free from an attention tensed state temporarily or over a long period of time in their driving operations.

When the events as described above frequently occur, it is assumed that the drivers operating the vehicles could be separated from a driving attention loop. For example, in spite of operating the vehicles, the drivers could think about things completely irrelevant to a forward side or surrounding recognition, fall asleep in an extreme case, make conversation with fellow passengers in an attitude completely different from an attitude for the forward recognition, or operate smart phones. In order to urgently return to a steering loop due to the occurrence of unexpected events such as forward accidents when the drivers are temporarily separated from a driving attention loop, it is indispensable for the drivers to instantaneously recover consciousness and grasp surrounding situations.

In short, as the partially or fully automated driving is introduced, the drivers become free from an attention tensed state temporarily or over a long time in driving operations. However, even at a driving time at which the drivers are not allowed to rely on the automated driving, the drivers are likely to take an action resulting in reduced attention due to their habituation.

There is a concern that driving actions separated from a driving loop requiring a high attention state occur frequently when partially automated driving becomes widespread with the aim of achieving fully automated driving. Since existing laws do not institutionally allow the separation of the drivers from vehicle control, driving operations at the time of restoring from automated driving have not become problems. However, as driving actions in which the drivers do not get involved in driving becomes widespread after the revision of institutions, it is assumed that reduced attention at the time of restoring to the driving actions is seen as a problem.

Unless technologies allowing vehicles to perform fully automated driving until completely stopping at destinations after departures are established, possibilities in which the drivers shift from the automated driving to manual driving remain until the end of driving. When shifting to manual driving, the drivers are required to recognize or understand surrounding environments and take over the driving and steering of the vehicles. If driving is shifted from the automated driving to the manual driving in a state in which the recognition or the understanding of surrounding environments or driving situations by the drivers is reduced when the drivers are sleepy-eyed or in a dream or the like, the drivers shift to incomplete steering to cause the risk of inducing serious accidents.

Therefore, when the drivers restore to the manual driving as the emergency response of the automated driving, the recovery state of a mental consciousness level at which the drivers recognize or grasp the surrounding environments is required to be confirmed on the side of a driving assistance system that performs automated driving control.

Existing systems that grasp the states of drivers with in-vehicle cameras are basically assumed to observe the drivers in their fixed attitude. The drivers are in the fixed attitude when focusing on driving, but are in a free style in which the attitudes or the heads of the drivers widely move when the driver are free from an attention tensed state. Therefore, it is considered that the existing systems are not suitable for observing the drivers free from the attention tensed state and hardly determine the manual driving ability of the drivers with high accuracy.

Patent Literature 4, according to its abstract, relates to a driver state monitoring (DSM) system that includes an image obtainment apparatus configured to obtain image information of a user driving a vehicle; a biological information obtainment unit configured to be worn on a specific portion of the user's body and to obtain biological information from the specific portion of the user's body; and a controller configured to detect a dangerous driving-state in which the user drives the vehicle, based on the image information and the biological information of the user.

Patent Literature 5, according to its abstract, relates to an image pickup device that has a plurality of image pickup regions, and is provided with: image pickup means for generating an image signal corresponding to an optical image incident on the image pickup regions; specific region detection means for detecting, on the basis of the image signal outputted from the image pickup means, a specific region of an image indicated by the image signal; and control means having a first control unit for exerting control so that an image pickup region on which the optical image corresponding to a specific region among the plurality of image pickup regions is incident is image-picked up under a first photographing condition and a second control unit for exerting control so that an image pickup region other than the image pickup region on which the optical image corresponding to the specific region among the plurality of image pickup regions is incident is image-picked up under a second photographing condition different from the first photographing condition.

Patent Literature 6, according to its abstract, relates to a Vehicle including a seat in which an occupant sits during use of the vehicle and a monitoring system for monitoring the occupant in the seat. The monitoring system includes sets of electric field antennas, each including at least one antenna, a control unit connected to the antenna sets and including selectors coupled to the antennas. The selectors are controlled by the control unit to obtain signals from one or more antennas serving as receiving antennas and one or more antennas serving as sending antennas. The control unit determines which combination of sending antenna(s) and receiving antenna(s) provides a strongest signal in an expected heartbeat range and/or expected respiration range of the occupant and then monitors this combination for changes and/or deviations from a normal range of heartbeats and/or respiration.

Patent Literature 7, according to its abstract, relates to a vehicle control apparatus that switches an operating condition of a vehicle to a manual operation when the vehicle reaches a preset initial switch position while an automatic operation is underway includes: a driver condition determination unit configured to determine whether or not a driver is in a manual operation acceptance condition; an evacuation space identification unit configured to identify an evacuation space provided on a path of the vehicle before the initial switch position on the basis of map information; and a switch position setting unit configured to set a switch position in which the operating condition of the vehicle is to be switched from the automatic operation to the manual operation, in a position between the vehicle and the evacuation space when the driver is not in the manual operation acceptance condition.

### Citation List

### Patent Literature

Patent Literature 1: Japanese Patent Application Laid-open No. 2010-262478
Patent Literature 2: Japanese Patent Application Laid-open No. 2015-127937
Patent Literature 3: Japanese Patent Application Laid-open No. 2015-226766
Patent Literature 4: US 2015/328985 A1
Patent Literature 5: JP 2014 179892 A
Patent Literature 6: WO 2015/174963 A1
Patent Literature 7: EP 2 979 914 A2

### Disclosure of Invention

### Technical Problem

The technology disclosed in the present specification has an object of providing an excellent image processing device and an image processing method capable of processing an image of a driver picked up by an in-vehicle camera. Solution to Problem

The technology disclosed in the present specification is achieved in consideration of the above problem. Therefore, there are provided an image processing device and an image processing method according to the independent claims.

### Advantageous Effects of Invention

According to the technology disclosed in the present specification, it is possible to provide an excellent image processing device and an image processing method capable of processing an image of a driver picked up by an in-vehicle camera.

Still other objects, characteristics, and advantages of the technology disclosed in the present specification will become apparent with more detailed descriptions based on the following embodiments and accompanying drawings.

### Brief Description of Drawings

[Fig. 1] Fig. 1 is a diagram for describing an image pickup operation by an in-vehicle camera 100 to which the technology disclosed in the present specification is applied.
[Fig. 2] Fig. 2 is a diagram showing a configuration example in which the portion of a capture frame selectively reads an image at high speed.
[Fig. 3] Fig. 3 is a diagram showing an example of the interface wiring diagram between ICs that realize high-speed reading.
[Fig. 4] Fig. 4 is a diagram showing a configuration example of a laminated image sensor in which a pixel region and a signal processing circuit region are laminated together.
[Fig. 5] Fig. 5 is a diagram showing a configuration example of a laminated image sensor in which a pixel region, a memory region, and a signal processing circuit region are laminated together.
[Fig. 6] Fig. 6 is a flowchart showing a processing procedure for acquiring a consciousness level at which a driver recognizes a target on the basis of an image picked up by an in-vehicle camera.
[Fig. 7] Fig. 7 is a diagram schematically showing the functional configuration of an image processing device 700 that acquires a consciousness level at which a driver recognizes a target on the basis of an image picked up by an in-vehicle camera.
[Fig. 8A] Fig. 8A is a flowchart showing a processing procedure (first half) for tracking the eyes of a driver and recognizing the consciousness level of the driver.
[Fig. 8B] Fig. 8B is a flowchart showing a processing procedure (second half) for tracking the eyes of the driver and recognizing the consciousness level of the driver.
[Fig. 9] Fig. 9 is a flowchart showing a processing procedure for tracking the saccade of the eyes at a high frame rate.
[Fig. 10A] Fig. 10A is a diagram summarizing respective processing (first half) performed simultaneously with the behavior of the driver.
[Fig. 10B] Fig. 10B is a diagram summarizing respective processing (second half) performed simultaneously with the behavior of the driver.
[Fig. 11] Fig. 11 is a diagram for describing an action taken by a driver to visually recognize a pedestrian around a traveling road when a vehicle steadily travels forward.
[Fig. 12] Fig. 12 is a diagram schematically showing the configuration of a man's visual field.
[Fig. 13] Fig. 13 is a diagram showing an example of the recognized visual information of a central visual field and surrounding visual fields when an observer sees a target in a stationary state.
[Fig. 14] Fig. 14 is a diagram for describing a sequence in which a man sees an object not existing in a central visual field.
[Fig. 15] Fig. 15 is a diagram showing an example of the actions of the visual line (eyes) and the head of a driver during the steady traveling of a vehicle.
[Fig. 16] Fig. 16 is a diagram showing an example of a sequence in which the driver sees an object not existing in the central visual field during the steady traveling of the vehicle.

### Mode(s) for Carrying Out the Invention

Hereinafter, embodiments of the technology disclosed in the present specification will be described in detail with reference to the drawings.

There could be a case that when a vehicle is in a driving mode such as a partially automated driving or a fully automated driving, the vehicle should restore to manual driving intervened by a driver as an emergency response. On this occasion, a control system responsible for the automated driving control of the vehicle should cause the vehicle to restore to the manual driving after confirming the recovery state of a mental consciousness level at which the driver recognizes and grasps an environment. Accordingly, a method for confirming the mental consciousness level of the driver is important.

For example, like a case in which a referee calls out to a knocked down player in a boxing bout, it is ideal to grasp a hand of a driver and confirm the squeezing back reaction of the driver or observe the stability of the visual line or the response of the driver since the recovery state of the consciousness of the driver can be exactly determined. However, according to a technological standard at the filing of the present application, it is difficult to realize such an interactive confirmation method.

According to a method for monitoring the response of a driver in response to an active input to the driver and determining a consciousness state, the consciousness state of the driver can be confirmed artificially like a boxing referee does.

However, according to a technological standard at the filing of the present specification, it is difficult to develop an inexpensive device that measures the brain waves of a driver in a vehicle during traveling in a non-contact fashion. In addition, in order to obtain brain waves examined by an EEG (electroencephalogram) or a MEG (magnetoencephalogram) examined by a MRI (magnetic resonance imaging) or the like, a large device is required. Such a large device can be used for a medical and academic purpose, but an ordinary man cannot use the device in a daily life. In short, a method for measuring brain waves to instantaneously confirm the determination ability of a driver is not practical.

In view of this, the present specification will disclose a technology by which the action of the visual line of a driver to intentionally grasp a surrounding environment is monitored to comparatively readily and effectively determine driver's will discrimination ability and momentarily and directly read the recovery state of a mental consciousness level at which the driver recognizes and grasps the surrounding environment.

First, the characteristics of the movement of the eyes of a man will be considered.

As a man's normal action for recognizing and determining a target, the man moves his/her eyes, head, and attitude in this order and causes the target to be placed in a central visual field where necessary when a conscious desire to see the target blurred in a surrounding visual field is created. Specifically, the eyes first rotate singly and catch the target around the central visual field to make the target seen in the central visual field only with the eye rotation. When the target exists in a direction in a range not coverable only with the eye rotation, the man follows the target with the eye rotation prior to the movement of the head or the posture and then rotates the head and the posture.

At this time, due to human (anatomical) characteristics in which the rotation inertia force of the eyes is really smaller than those of the body and the head, the eyes can be instantaneously oriented in an attention direction, and then the head or the like rotates. Accordingly, as the movement of the eyes to see an object, the eyes once instantaneously rotate in the attention direction and reversely rotate with respect to the head so as to cancel the following rotation of the head.

Meanwhile, eyes have advanced to perform eye movement called saccade or microsaccade to rotate quickly and little by little even when a visual line seems to stay at the same place.

The saccade and the microsaccade are quick and impulsive eye movement performed peculiarly when a man is in an awakening state. Specifically, the eye movement is quick and fine eye movement composed of awkward and discontinuous rotation acceleration and wiggling movement in which the eyes stop after being oriented in one direction. As one reason for performing such a type of eye movement, it becomes easier for the man to acquire the same projection image and recognize a visual event for a certain period with the same rod cell or cone cell when seeing a target in a certain direction in a stationary state to prevent the flow of an image rather than continuously rotating the eyes at all times. In addition, as another reason, when the eyes are continuously exposed to the same brightness of a certain level or more, visual light-receiving sensitivity reduces, which in turn makes it impossible to acquire the target. After the recognition of the target or after the cancellation of the rotation of the head, the microsaccade is repeatedly performed around a visual line direction.

In addition, an image in a real world space projected on eyegrounds when the eyes temporarily stop during the discontinuous eye movement and an image recognized by the man in the brain are not necessarily recognized as absolute positions. Even if there is a directional deviation with respect to a target seen by the saccade of the eyes, a projected optical image captured by the retina is taken over to a primary visual area, a visual association area, and a high-order association area while being transmitted from the eyes to the brain. By referring to the last high-order association area, human memory, or the like, the man intellectually recognizes the target.

The absolute coordinates of the real world space projected on the retina and recognition coordinates recognized in the brain are momentarily corrected in a recognition operation. The recognition correction is an event occurring in the consciousness of the man and thus cannot be directly observed. The validity of the hypothesis is supported as follows. For example, when the head is rotated with a visual line assumed as an axis, the eyes unconsciously perform discontinuous rotation movement to cancel out the rotation. However, the rotation amount of the eyes at which a superior oblique muscle or an inferior oblique muscle can continuously rotate is small. In addition, if the rotation amount of the eyes exceeds their followable rotation amount, the eyes restore to an initial non-rotation state in the high-speed saccade and repeatedly cancel out the rotation of the head again to release the stress of the superior oblique muscle or the inferior oblique muscle that drives the rotation of the eyes. However, on this occasion, the man does not feel consciousness in which the recognized outer world of the world space is rotated. That is, even if the eyes rotate in a way in which an image of the real world space projected on the retina when seen from the coordinates of the eyes is discontinuous, such high-speed restoration rotation is not recognized in perception and consciousness.

That is, the correction between a human visual line direction and an actual recognition direction associated with the saccade or the microsaccade of the eyes is performed at all times in a process in which the real world space seen by the eyes (or projected on the retina) is transmitted from the primary visual area to the visual association area. Consequently, an exact consciousness state cannot be necessarily discriminated unless the correlation between the visual line and the world space is taken from the dynamic analysis of the saccade or the microsaccade.

From the above reason, it cannot be said that accuracy obtained when the eye movement is caught at an update rate of, for example, 60 fps or so by an ordinary camera to analyze tracking movement is not sufficient for quickly determining the consciousness level of the driver. Even with a high-accuracy visual line recognition system, its recognition and detection accuracy in a visual line direction is 0.5 degree or so at the most in measurement under an ideal environment. Generally, the reason why absolute physical measurement accuracy by measurement is 0.5 degree or so as described above is based on the hypothesis that an absolute projection position on sensory nerve cells where a sensory absolute address is projected on the retina is always matched. It is assumed that since an actual recognition address is interpolated and corrected by a sensory area or the like, an error occurs due to the deviation between a physical direction and a recognition direction.

If the saccade or the microsaccade of the eyes can be tracked at high speed and the eyes can be always monitored in a free style state regardless of the attitude of a driver, it becomes possible to acquire a consciousness level at which the driver recognizes a world space and a target with certain high accuracy.

Next, the visual area eye movement of a man will be considered.

A mechanism in which a man's visual sense recognizes the surrounding situation of an environment in detail is not realized in such a way that a visual line is simply oriented in the direction of a specific target desired to be perceived in detail, and that the direction of a central visual field in which the target can be seen with high precision is found on absolute spatial coordinates.

Eyes rotate in one stretch to perceive a target in detail. When the target is separated from a central visual field, a head rotates to compensate for the narrow range of the eye rotation. On this occasion, the eyes do not rotate in sync with the action of the head as the saccade of the eyes, but the eyes rotate and turn in an objective direction at one stretch. Along with the movement of the head, the eyes capture the target in the direction of the central visual field. The eyes move to adjust the central visual field to the target in such a way as to cancel out the movement of the head at a level at which the eyes are not forced to move in terms of a human body structure. After that, the eyes start a recognition operation to acquire high-precision information.

At this time, the eyes do not rotate and move to completely cancel out the rotation of the head one on one. However, the eyes repeatedly perform saccade composed of wiggling canceling-out rotation and are configured to follow a direction in which the discontinuous and rapid eyes move by several steps to cancel out the movement. Reflection of a virtual image on the retina in a real space associated with each rotation of the eyes is not recognized on the absolute coordinates of a projection surface, but the deviation of a projected position is corrected to repeatedly perform the interpretation of a position in the brain unconsciously. Therefore, generally, the direction of visual line recognition obtained by the analysis of the movement of the eyes cannot be exactly calculated as expected.

However, in many cases, the cause of the error is linked to the fact that the brain and recognition coordinates at a projected point in a physical engineering real space are momentarily corrected. In addition, a period at which a man sees a target in a central visual field to complete the recognition of the target is determined on the basis of various factors such as the recognition experience of the man under the same condition and is greatly associated with the awakening degree of the man.

Accordingly, by exactly analyzing eye movement when a driver recognizes an environment during traveling and the delayed recognition of the direction information of the spatial arrangement of an object existing in a real space, it is expected to grasp the ability to recover an awakening state by the driver with respect to driving with high accuracy and at an early time.

Particularly, in the case of a vehicle in which fully automated driving is not realized in an emergency, partially automated driving, or the like, it is demanded to perform a sequence for transferring control to a driver at an early time in an emergency. However, if a driving operation is taken over to the driver who incompletely recognizes a situation, a driving malfunction accompanying a situation grasping error cannot be consequently completely avoided. In addition, if vehicle steering right is taken over to the driver in a situation in which the driver is required to make a determination to perform an operation while insufficiently grasping a surrounding situation, a normal determination and action sequence consequently does not work since the driver is in a panic state due to a situation beyond the determination ability of the brain of the driver. It is known that once the driver is in a panic state, a nerve recognition network responsible for a recognition and determination action becomes completely jammed and the whole action stops until the driver affords to perform thinking and action even if the driver visually sees a risky state.

Therefore, when it is possible to successively monitor continuous tracking with respect to the visual information to which the attention visual line of the driver is likely to move and which is obtained from the visual line action of the driver traveling on a highway and a saliency map or obstacle information, the realization of the effective confirmation of the state of the driver at each time becomes possible.

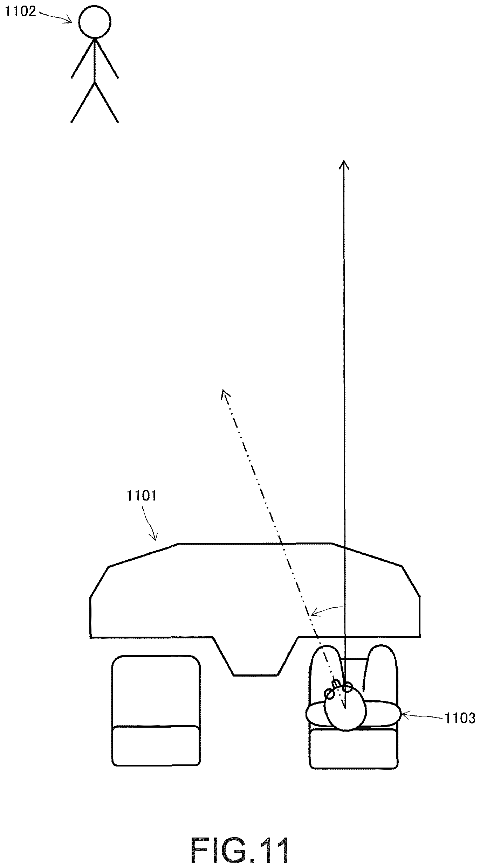

An action taken by a driver 1103 to visually recognize a pedestrian 1102 around a traveling road when a vehicle 1101 steadily travels forward as shown in Fig. 11 will be considered.

When the pedestrian 1102 seen by the driver 1103 with his/her attention visual point moving is deviated from a visual field angle unique to the driver 1103, the driver 1103 comprehensively performs turning confirmation by using head rotation and eye rotation together. A sequence for determining a situation with the visual line movement of a man is completed when the characteristic content of a target object is recognized by the visual line movement. When the object does not exist in a central visual field from the beginning, the man turns in a direction in which the object is likely to exist. With the head rotation and physical body movement where necessary, the man causes the object to move to the central visual field on the retina. The above situation determination sequence is completed at a point at which information enough to make a determination is acquired in a sensory area. The above fact can be understood by the analysis of the movement of the visual lines of an ordinary driver and a driver required to make a quick determination in an occupational auto race or the like. That is, a driver required to make a quick determination in an occupational auto race or the like has a really short visual fixation time at the movement destination of a visual line on a saliency map and moves the visual line to a large number of targets at high speed and frequently overlooks the same to make an exact determination at all times. Consequently, the driver repeatedly moves the visual line to such a degree that he/she does not have a time to fix eyes on a separate target. However, it is assumed that the driver reaches a perceptual threshold required to make a determination even if he/she does not perform visual fixation to stop the visual line. This indicates that the driver can make a determination even with a less stimulus on the basis of an experience.

The driver performs the saccade of eyes, the rotation of a head, and the rotation and the movement of a physical body in this order at low speed. In addition, a range coverable by the saccade of the eyes is limited.

Note that in recognition in terms of brain science, a positional deviation or rotation with respect to the strict position of two-dimensional information in a projected real space is moved and interpolated to be recognized on cranial nerves in such a way as to interpolate the projection of the central visual field of a target on the retina of the physical eyes. Thus, the driver does not consciously feel the saccade including the wiggling rotation of the actual eyes.

According to conventional visual line tracking, it has been assumed that a recognition target to be visually perceived by an observed person can be exactly grasped with its physical measurement accuracy if it is possible to detect the exact physical direction of the eyes.

However, even if the eyes wiggle and discontinuously visually observe a target, recognition nerves actually interpret a three-dimensional outer world by performing interpolation from the storage arrangement of the target. For example, even if a head rotates, an image of a space consciously felt does not rotate. In addition, slight horizontal rotation is felt when a head rotates right and left at high speed, but a horizontal space remains horizontal.

When moving the visual line in a horizontal direction, the eyes first perform the saccade to move to a place having large brightness, a large chroma position, or a large temporal change in a surrounding visual field to capture a target so that the direction of the target enters a central visual field. When the eyes are oriented in the direction without having any difficulty, the eyes depend only on their rotational movement to try to catch the direction of the target. When the target exists outside the range of moderate eye rotation, the head also starts rotating simultaneously with the movement of the eyes and rotates. Where necessary, a physical body also rotates and moves.

A head is heavier than eyes themselves. Therefore, the rapid rotation of the head results in a burden on a neck and is not frequently performed in a situation other than a special situation in a short time such as a sporting event or the like.

In most cases, the movement of the visual line of a driver when a vehicle normally travels straight (on the basis of manual driving) can be covered only by the rotation of eyes. Therefore, generally, a tracking device considering the large movement of a head to recognize the state of a driver is not necessarily required.

However, in a case in which a driver does not necessarily see a forward side in an ordinary attitude with the widespread of automated driving, a case in which the screen of a CMS (camera monitor system) is observed, or the like, it becomes difficult to instantaneously grasp the consciousness state of the driver unless the movement of a head and eyes is not tracked widely and in an integrated fashion. Therefore, it is difficult to perform processing for making a determination as to whether automated driving is shifted to manual driving in several seconds to one second or less.

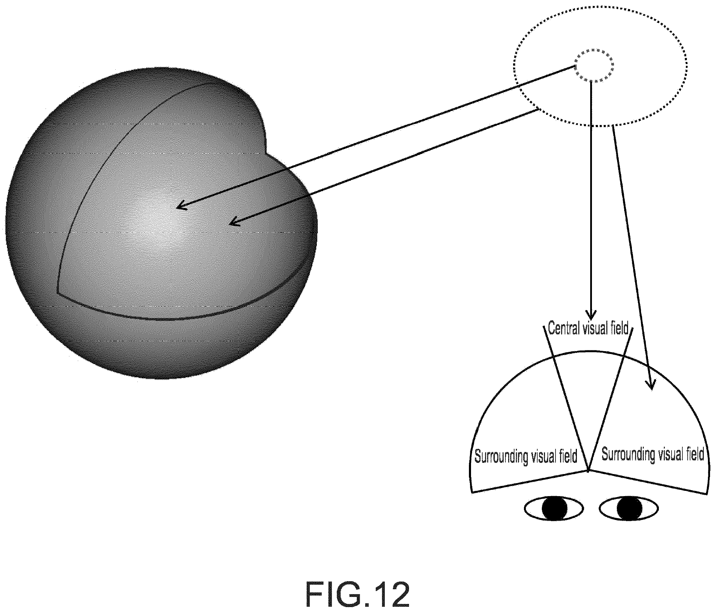

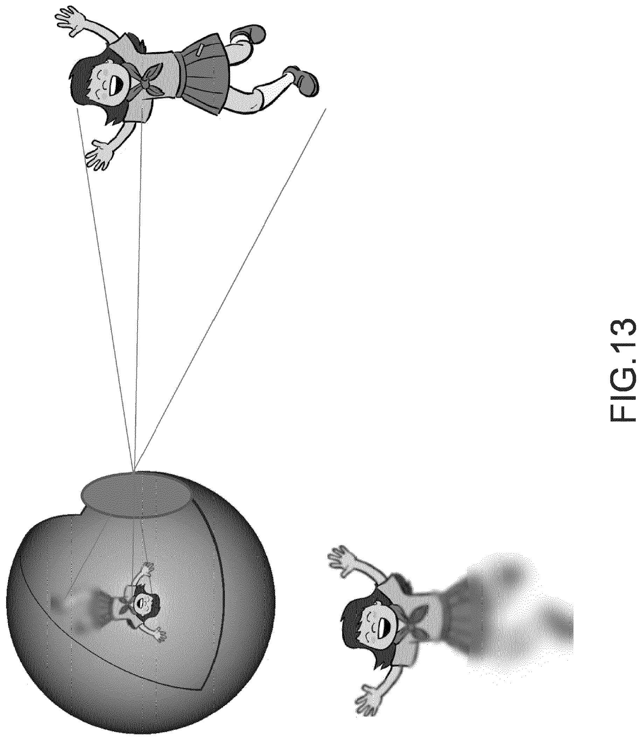

Fig. 12 schematically shows the configuration of a man's visual field. As shown in the figure, a man has a maximum visual field angle of about 200 degrees in a horizontal direction, and the visual field is composed of a central visual field in which a target in a front direction can be seen at high resolution and surrounding visual fields on right and left sides in which the movement of the target is mainly detected at low resolution. In addition, Fig. 13 shows an example of the recognized visual information of a central visual field and surrounding visual fields when an observer (driver) sees a target in a stationary state. The target is seen in a blurred state as the observer sees the target in the surrounding visual fields outside the central visual field in which the observer performs visual fixation. In addition, the target is seen in a desaturated state as the observer sees the target in the surrounding visual fields.

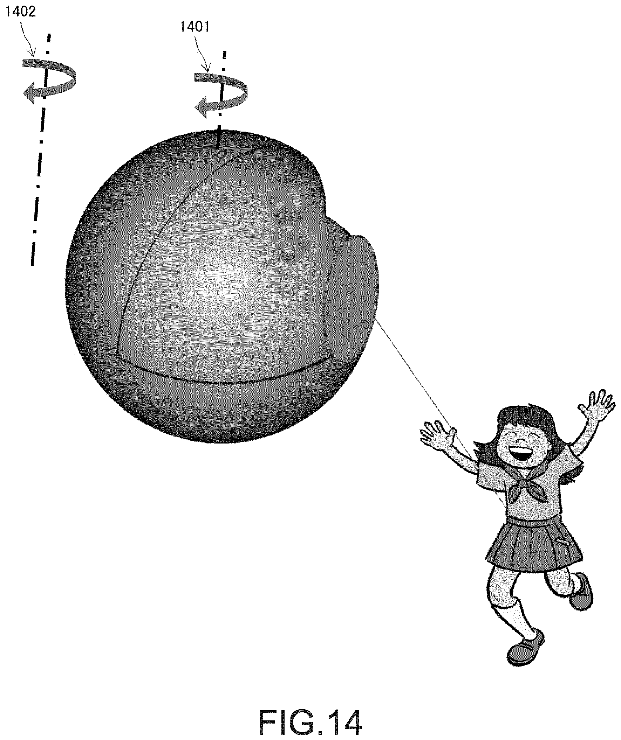

A sequence in a case in which a man (driver) sees an object not existing in a central visual field will be considered with reference to Fig. 14. In the sequence, the man moves the central visual field in a specific direction according to a change or a will to grasp a situation.

The sequence in which the man sees the object not existing in the central visual field includes quick eye rotation denoted by reference numeral 1401 and head rotation following eyes denoted by reference numeral 1402. When the head rotation catches up with a target, the eyes relatively reversely rotate with respect to a head to cancel out the head rotation. Note that when the central visual field approaches and passes through the target in a turning process, the eyes shift to cancellation with respect to the head rotation, i.e., rotation for visual field stability so-called compensatory eye movement. In addition, when the target does not exist at infinity, so-called vergence eye movement corresponding to the distances of compound eyes is performed in parallel.

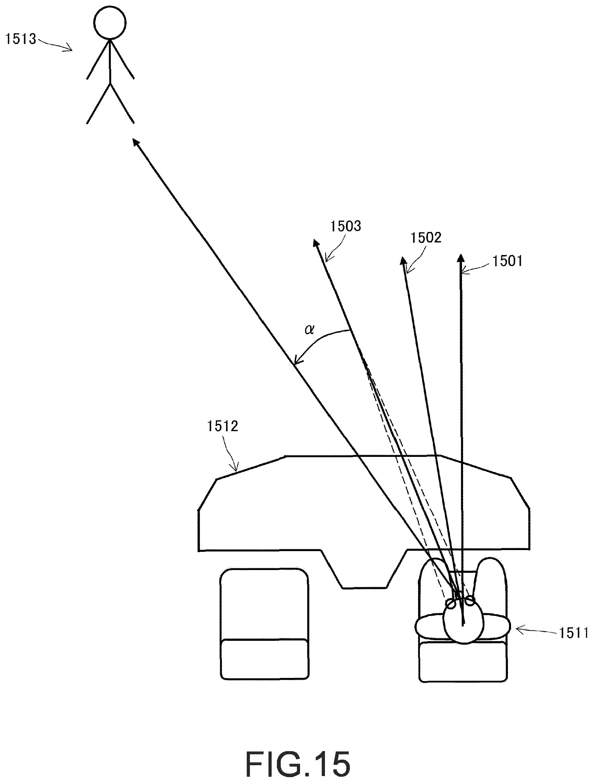

The actions of the visual line (eyes) and a head of a driver during the steady traveling of a vehicle will be described with reference to Fig. 15. In the figure, reference numeral 1501 denotes a forward side, i.e., the traveling direction of a vehicle 1512. In addition, reference numeral 1502 denotes the direction of the head of a driver 1511, and the head rotates by a horizontal rotation angle with respect to the traveling direction 1501 of the vehicle. In addition, reference numeral 1503 denotes the direction of the visual line of the driver, and the visual line further rotates by a horizontal rotation angle with respect to the head. Further, a range of ± 1.5 degree relative to the direction of the visual line 1503 becomes a central visual field.

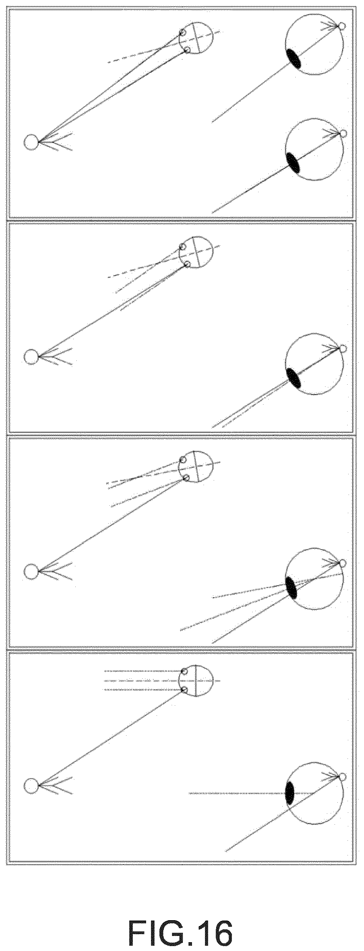

A sequence in which the driver sees an object not existing in the central visual field during the steady traveling of the vehicle is shown in, for example, Fig. 16 and will be described as follows. Here, as shown in Fig. 15, a case in which a target such as a pedestrian 1513 appears in a surrounding visual field in which the visual line 1503 of the driver further rotates by an angle α will be assumed.
(0) In the steady traveling, the driver drives the vehicle with his/her visual line oriented to a forward side.
(1) Here, it is assumed that attention is drawn in a direction other than the forward side. For example, when a pedestrian or the like that could be an obstacle appears and when stimulated by dynamic visual power from an outer world, the driver moves the visual line on the basis of his/her will to turn at an intersection or confirm a door mirror.
(2) The driver starts rotating eyes so as to cause a target desired to be confirmed to fall within the central visual field. When the visual field angle of the target is outside the range of the movement of the visual line, the driver then rotates his/her head or the attitude of his/her human body.
(3) On this occasion, the driver performs eye movement, head rotation, and attitude rotation in an order of a small load to cause the target to enter the central visual field.
(4) It has been known that the movement of the eye rotation is composed of wiggling saccade in that the eye rotation has the smallest load and the eyes can move at high speed.
(5) The driver starts rotating the eyes at high speed in the direction of the target. In a state in which the eye rotation is insufficient as a turning amount or a state in which there is a difficulty in the action range of the eyes, the driver starts an operation to visually capture the target in the central visual field when the target enters in a range coverable by the eye rotation in the middle of head movement compensated by the head rotation or the attitude movement. Then, in order to understand the event of the target, the driver adjusts the focal points of his/her eyes to start vergence eye movement. Note that when the target moderately moves from the central visual field to its outside, the eyes instantaneously turn in the direction of the target. After sufficient turning is ensured as the head rotation follows, the eyes reversely rotate in a direction in which their rotation with respect to the head is cancelled out, i.e., the driver performs compensatory eye movement to search for a secured visual line.
(6) The above series of turning actions and a recognition operation in the brain advance at high speed and in parallel. The recognition operation does not start after the direction of the eyes is stabilized with respect to the target, but the eyes perceptually correct and interpret miniature eye movement during the wiggling saccade of the eyes to advance the recognition of a situation.
(7) That is, the driver acting as an outer world observer does not interpret a real world space in which the target is projected on the absolute coordinates of the central visual field. However, in a recognition action, the driver performs the recognition and interprets a space while correcting a deviation occurring when the eyes minutely rotate due to saccade. Therefore, even if the physical absolute direction of the eyes is detected, the observer does not necessarily recognize and understand the direction.
(8) As the consciousness of the recognition action, it is presumed that an anatomical eye direction completely matches a recognition direction at the same time. As an exemplification, it is supported by environment recognition that while the eyes slightly move to cancel out moderate rotation with their rotation compensating for the head rotation and rotate with the saccade after exceeding the range of the eye rotation when the head rotates with eye lines serving as axes, the rotation is not recognized at all and a real space does not rotate but is in a stationary state.
(9) When the direction of the central visual field within a range coverable by the eye rotation is established in the middle of the head movement (before the rotation is completed), the driver acting as an observer gazes or fixes the eyes on the target entering the central visual field to visually start capturing. Then, the driver adjusts the positions of the focal points of the eyes to understand the event of the target, transmits much more and detailed information to a visual field and a visual association area, and identifies the information with memory in terms of a concept.

For example, as the driver catches a standing child with the central visual field and sees the details of the child, the driver recognizes the states of the child, i.e., the driver recognizes the color of clothing, a state in which the child raises a hand, a state in which the child wears student-like clothing and gives a smile. In addition, the driver recognizes who the child is if the driver knows the child. Such perceiving and recognizing actions are advanced in order, or some of the actions are advanced in parallel.

Meanwhile, the driver follows the target at high speed with the saccade. Particularly, when the target starts entering the central visual field, the driver adjusts the focal points of the eyes in parallel to start the vergence eye movement. In addition, the driver advances the head rotation until the attitude of the head is set in a relaxed position, and then decelerates and stops the rotation. When a direction in which the driver is aware of visual recognition to a certain degree is established, the eyes then advance the recognition while repeatedly performing the microsaccade in the vicinity of the direction.

In the case of driving an automobile, a driver does not gaze at a specific target with his/her visual line completely fixed to the target when traveling while grasping many environments around the vehicle. When recognition for making a determination of driving steering about a target seen in a certain visual line direction reaches a required level, the driver unconsciously or consciously starts saccade, head movement, or the like in another direction in which recognition is desired to be performed to grasp a target with higher priority.

The above series of eye movement relates to confirmation as to whether a driver is in an awakening state in which the recognition of a surrounding environment that could have influence on the driving of the driver is prioritized. When eyes turn in conjunction with the direction of the saliency map of a real world space in their searching action of the saccade, the driver is associated with a state in which he/she is highly conscious of the driving. The saliency map is a two-dimensional distribution map in which a direction attracting driver's attention due to the visual characteristics of a target is analyzed mechanically and arithmetically.

By observing eye movement continuously and endlessly occurring in a time-series fashion like saccade rather than observing the state of eyes staying in a stationary state, it is possible to discriminate the consciousness state of a driver. If the eye movement of the driver continuously and endlessly occurring in a time-series fashion can be continuously observed in a situation in which the driver in partially or fully automated driving is in a free style state and a head widely moves, it becomes possible to momentarily track the consciousness state of the driver without attaching an electrode or a wearable item to the driver.

If the saccade or the microsaccade of eyes can be tracked at high speed at all times and a driver can be monitored in a free style state regardless of the attitude of the driver, it becomes possible to acquire a consciousness level at which the driver recognizes a world space and a target with certain high accuracy.

In a case in which the saccade or the microsaccade is applied to processing for determining the consciousness state of a driver when partially or fully automated driving is shifted to manual driving, it is assumed that the driver is in a free style state in which the attitude or the head of the driver moves in a wider range compared with a case in which the driver is in an intensive driving attitude. In order to realize the tracking of the consciousness state of the driver in a wider range and with higher accuracy, it is necessary to continuously capture the head position of the driver and track the saccade of eyes in a wide visual field angle.

In order to realize the tracking of the visual line of a driver in a wide range, a device that mechanically follows the head of the driver detected by a camera at all times is assumed. However, if the reliability of such a mechanical tracking device is not really high, it is necessary to repeatedly repair or perform maintenance of the device in the life cycle of a vehicle. In addition, in order to prevent the generation of an accidental passenger injuring factor established as a safety standard, it is necessary to take many avoidance responses to reduce the mechanical injuring factor.

In addition, a device that is attached to the head of a subject to measure eye movement has been proposed (see, for example, Patent Literature 3), but an attachment load is generated. A driver is required to continuously wear the device on a head even in partially or fully automated driving, which is cumbersome and does not substantially give a sense of liberation even in automated driving. In addition, when a driver wears the device on a head in manual driving, the device becomes cumbersome for the driver and at the same time blocks a driver's vision.

Technologies for recognizing visual lines have been frequently proposed before the filing of the present application. However, since eyes move at really high speed in saccade or microsaccade, the detection of the eyes is performed in a way different from that of the eyes in a stopped state. In order to detect the quick saccade or the microsaccade, it is necessary to capture and analyze eye movement with a camera having a high frame rate. For example, it cannot be said that a frame rate of 60 fps or so is not sufficient for quickly discriminating the consciousness level of a driver. Here, when a camera having a high frame rate is used, a data rate increases. When a sensor that performs visual line detection or the like transfers data to a subsequent signal processing processor via a transmission path composed of long wiring, new problems occur in which energy radiation corresponding to a transmission loss is generated and many thermal and electromagnetic radiative energy losses are induced. In addition, in the signal reading system of a general image sensor, an energy loss increases to output all images from one reading path at a fixed rate. Further, since it not easy to respond to such an energy loss, a circuit is required to be driven with a lower frequency, which results in a difficulty in detecting a visual line with high efficiency.

In view of this, the present specification proposes a technology for catching the attitude or the head of a driver at a wide visual angle using one or more statically installed (fixed) cameras without mechanically following functions and capturing the saccade of eyes at high speed for an image of the positions of the eyes.

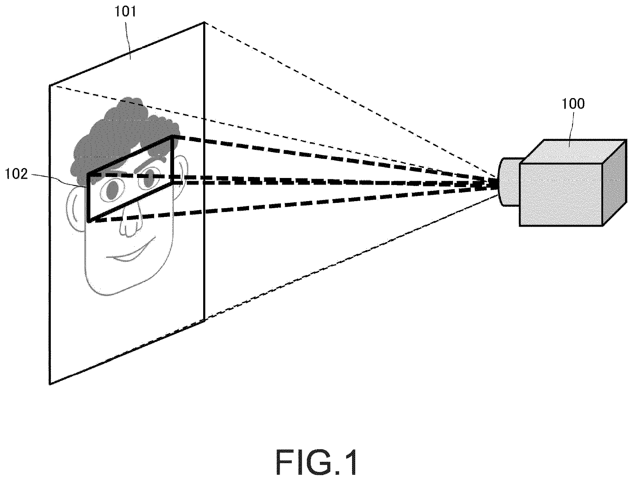

The outline of the technology disclosed in the present specification will be described with reference to Fig. 1.

An in-vehicle camera (so-called a drive monitor camera or the like) 100 picks up an image of a driver seated on a driver's seat. Note that a vehicle is switched between an automated driving mode (including both partially automated driving and fully automated driving) and a manual driving mode, and that the driver does not always take an intensive driving attitude but is assumed to be in a free style state in which a driver's attitude or head moves in a wide range since the driver is free from an attention tensed state. Accordingly, the in-vehicle camera 100 has a visual field angle of a wide-range area denoted by reference numeral 101. In order to detect quick saccade or microsaccade, it is necessary to capture the eyes of the driver and their detailed movement at a high frame rate of, for example, 1000 fps or so. However, since the image pickup area 101 covers a wide range, the in-vehicle camera 100 is allowed to pick up an image of the driver at, for example, 60 fps or so in the wide-range area 101 in consideration of a technological problem such as an energy loss due to a transmission loss caused by the transmission of an image pickup signal to a subsequent-stage signal processing processor when the in-vehicle camera 100 is separately provided and thus the occurrence of electrode unnecessary radiation noise as its energy release.

By picking up an image in the wide-range area 101, it is possible to nearly undoubtedly capture the head of the driver and track the positions of the eyes of the driver even if the driver is in the free style state.

Next, the picked-up image of the wide-range area 101 is analyzed to determine a narrow capture frame 102 corresponding to the eyes of the driver. Since the driver is in the free style state, the position of the capture frame 102 is assumed to move momentarily. Therefore, it is preferable to actively correct the position of the capture frame 102 at intermediate speed.

Then, inside the capture frame 102, picking up an image of the eyes at high speed of 1000 fps or so and high-speed image recognition are performed to realize the tracking of the saccade of the eyes.

In short, an image of a wide eyelips 101 is picked up by the in-vehicle camera 100 at low resolution and eye positions grasped in the image analysis are corrected for each frame while local high-speed processing is performed on the eye positions in parallel, whereby the tracking of the saccade among eye movement is realized. That is, the active local high-speed eye tracking is performed only on the capture frame 102 in the image picked up by the in-vehicle camera 100, whereby it becomes possible to monitor the saccade of the eye movement without causing an attachment load.

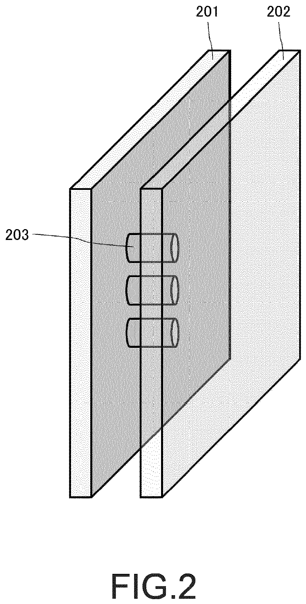

For example, as shown in Fig. 2, a logical IC (Integrated Circuit) 202 that performs image recognition processing is bonded to a (normal) image pickup element 201 so that only the image data of a partial image (that is, the capture frame 102) including the eye positions is selectively transmitted at high speed between the ICs. Thus, interface wiring 203 between the image pickup element 201 and the image recognition processing IC 202 can be minimized as silicon penetration electrodes (through-silicon vias, TSVs) and the partial image of the capture frame 102 can be selectively read at a high frame rate with a reduction in the reception load of the image recognition processing IC 202. Consequently, even if the driver is in the free style state in which the position of the head of the driver is not fixed, it becomes possible to track the eye positions and monitor the saccade of the eye movement at all times.

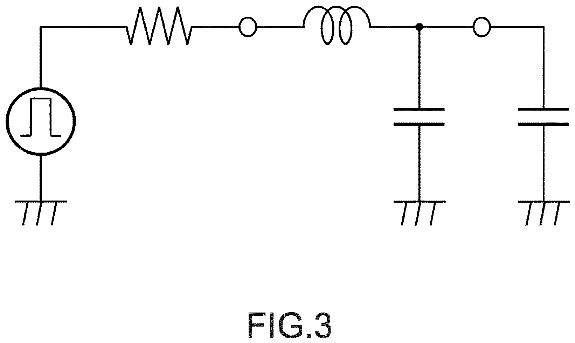

Fig. 3 schematically shows an example of an impedance factor parasitic on an interface wiring diagram between the image pickup element 201 and the image recognition processing IC 202. The image pickup element 201 and the image recognition processing IC 202 are bonded to each other by the silicon penetration electrodes as shown in Fig. 2 to minimize the wiring length of the interface wiring, and the parasitic impedance is reduced to make it possible to remarkably reduce an unnecessary wiring load causing a noise source. Thus, a further high-speed local image analysis is made possible, which brings about double advantages.

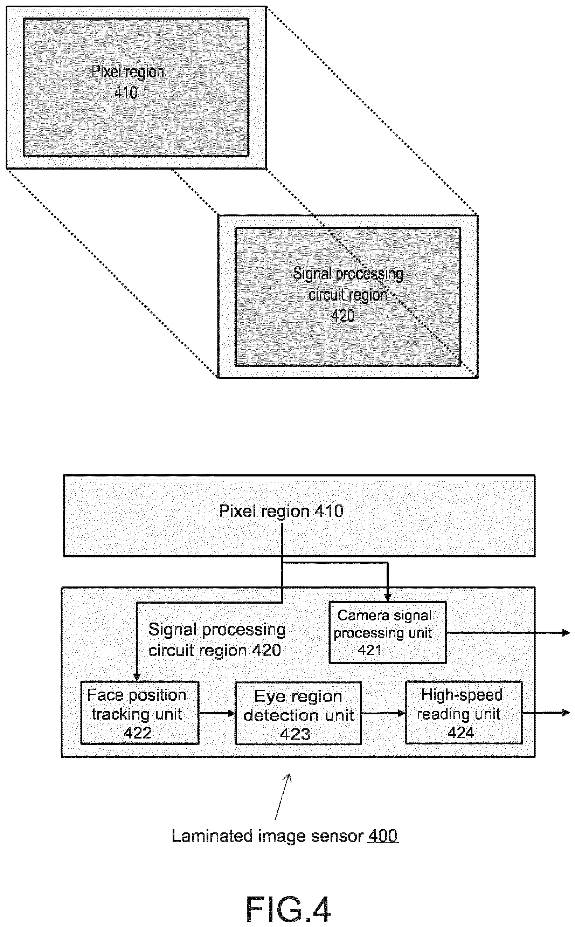

Fig. 4 shows a configuration example of a laminated image sensor 400 in which a pixel region 410 and a signal processing circuit region 420 are laminated together, the laminated image sensor 400 being capable of selectively transmitting the image data of a partial image at high speed.

A camera signal processing unit 421 reads the image data of the whole region of the pixel region 410 at a low frame rate of, for example, 60 fps or so to perform normal camera signal processing.

Meanwhile, a face position tracking unit 422 tracks the face region of a driver from the whole region of the pixel region 410. An eye region detection unit 423 detects the eyes of the driver from the face region tracked by the face position tracking unit 422 to determine a capture frame (described above) including the image data of a partial image of both eyes. Then, a high-speed reading unit 424 selectively reads the image data of the partial image of the determined capture frame at high speed.

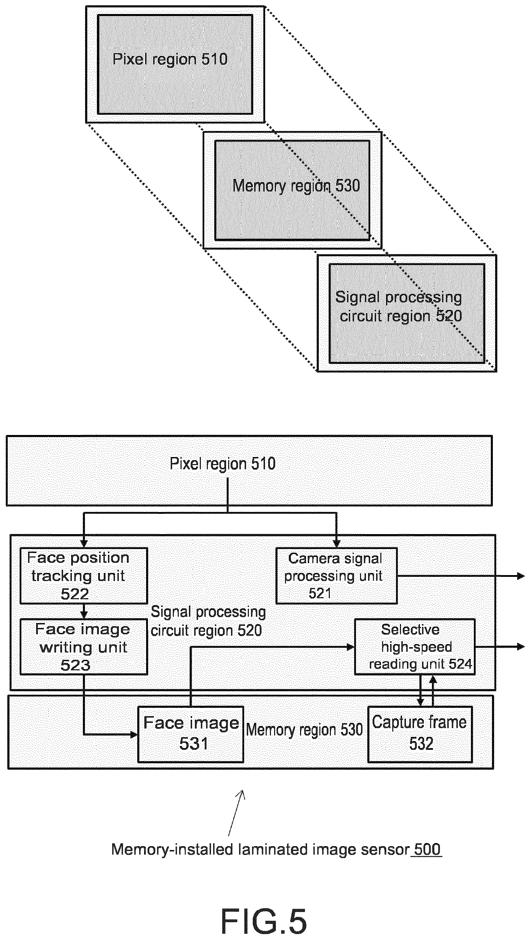

In addition, Fig. 5 shows a configuration example of a memory-installed laminated image sensor 500 in which a pixel region 510, a signal processing circuit region 520, and a memory region 530 are laminated to each other. The memory-installed laminated image sensor 500 can selectively transmit the image data of a partial image at high speed. The memory-installed laminated image sensor 500 temporarily stores the data of an image picked up at a high frame rate of 1000 fps or so in the memory region 530. Then, the stored image data is read at a desired frame rate.

A camera signal processing unit 521 reads the image data of the whole region of the pixel region 510 at a low frame rate of, for example, 60 fps or so to perform normal camera signal processing. Part of the image data captured at 1000 fps is read at a low frame rate of 60 fps or so, whereby the processing load of camera signal processing can be reduced.

In addition, the image data of the whole region of the pixel region 510 is temporarily stored in the memory region 530. A face position tracking unit 522 tracks the face region of a driver from the whole region of the pixel region 510, and a face image writing unit 523 writes a face image 531 in the memory region 530. Then, when detecting the eyes of the driver from the face image 531 and determining a capture frame, a selective high-speed reading unit 524 selectively reads the image data of the capture frame 532 at high speed. For example, the image data of the capture frame 532 is read at a high frame rate of 1000 fps or so the same as that of initially-captured image data. By the employment of such a configuration, the signal processing of only the capture frame 532 can be performed. Therefore, even if image data is read at a high frame rate of 1000 fps or so, a signal processing load can be reduced. In addition, by the use of the memory-installed laminated image sensor 500, it becomes possible to read images at different frame rates with one image sensor. Compared with a case in which two image sensors are used, a reduction in the number of components and a reduction in cost can be attained.

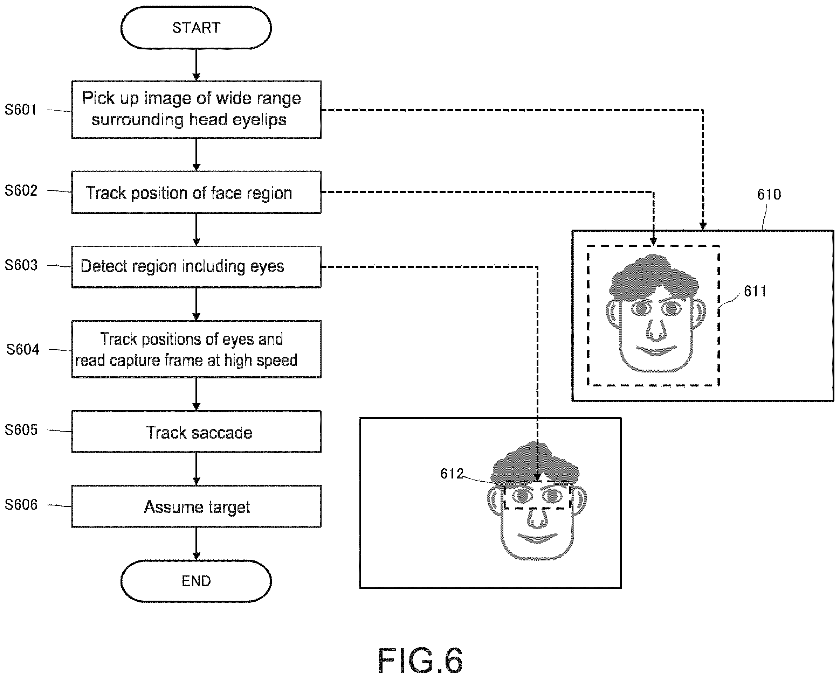

Fig. 6 shows, in the form of a flowchart, a processing procedure for acquiring a consciousness level at which a driver assumed to be in a free style state recognizes a target on the basis of an image picked up by an in-vehicle camera. As the in-vehicle camera, the laminated image sensor 400 shown in Fig. 4 or the laminated image sensor 500 shown in Fig. 5 is, for example, assumed.

First, an image of a wide range 610 surrounding the head eyelips of the driver is picked up by the in-vehicle camera (driver monitor camera) at a low speed of, for example, 60 fps or so and at low resolution (step S601).

Next, the position of a face region 611 of the driver is tracked from the whole region of the image picked up by the in-vehicle camera (step S602).

Then, a region including the eyes of the driver is detected from the tracked face region (step S603). The tracking of the positions of the eyes is started, and the image data of a partial image of a capture frame 612 is selectively read at high speed of, for example, 1000 fps or so while the position of the capture frame 612 including both the eyes is momentarily corrected (step S604).

Next, the saccade of the eye movement of the driver is tracked using the partial image read at high speed (step S605). Note that since the saccade includes personal characteristics, the tracking of the saccade may be performed on the basis of a result learned for each driver acting as a subject (as will be described later) .

Then, a target to be observed by the driver is assumed on the basis of the tracking of the eyes (step S606), and a consciousness level at which the driver recognizes the target, i.e., the awakening degree of the driver is further assumed.

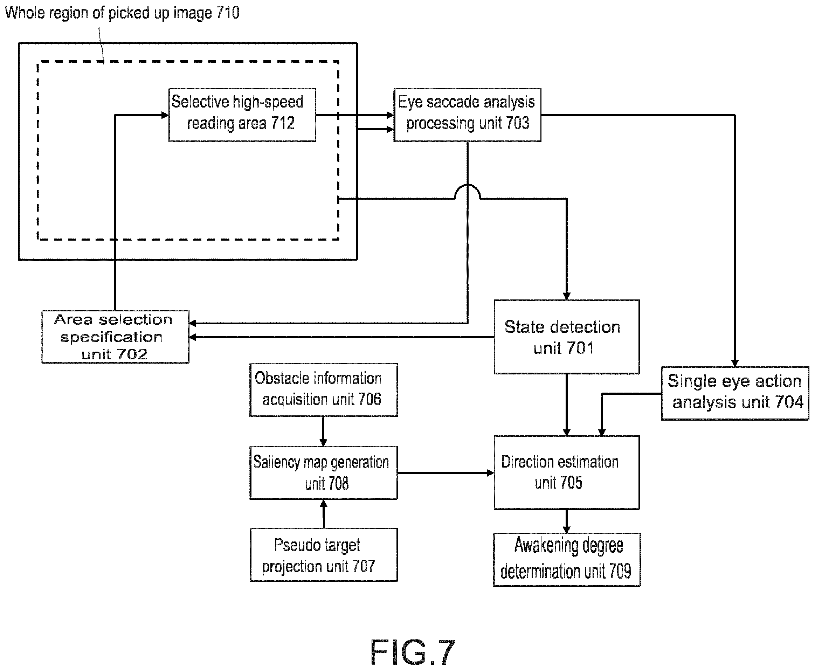

Fig. 7 schematically shows the functional configuration of an image processing device 700 that acquires a consciousness level at which a driver recognizes a target on the basis of an image picked up by an in-vehicle camera. Respective function modules 701 to 709 can be configured as dedicated hardware circuits but can be realized as a sequence obtained when a CPU (Central Processing Unit) performs a prescribed program code. Hereinafter, the operations of the respective units will be described.

The in-vehicle camera is assumed to pick up an image of a range surrounding the head eyelips of the driver. A state detection unit 701 detects the states of the head and the upper body of the driver from a whole region 710 of the image picked up by the in-vehicle camera and tracks the face region of the driver.

An area selection specification unit 702 specifies, from the face region tracked by the state detection unit 701, a selective high-speed reading area (area equivalent to the above capture frame) 712 in which selective high-speed reading should be performed.

An eye saccade analysis processing unit 703 performs selective high-speed reading of, for example, 1000 fps or so only on a partial image of the specified selective high-speed reading area 712 among the whole region 710 of the image picked up by the in-vehicle camera.

A single eye action analysis unit 704 analyzes the eye movement of saccade for each of the right and left eyes of the driver in the image read by the eye saccade analysis processing unit 703. For example, the single eye action analysis unit 704 may determine a dominant eye of the driver and analyze the saccade of only the dominant eye. In addition, since it has been known that eye movement becomes unstable due to diseases, it is desirable to conduct an evaluation in consideration of such characteristics unique to the driver. Moreover, since it has been known that one of the right and left eyes is more perceivable as a dominant eye, one of the eyes more effective for determining an awakening state may be used for the analysis.

Note that since the saccade includes personal characteristics, the single eye action analysis unit 704 may track the saccade on the basis of a result learned for each driver acting as a subject (as will be described later).

A direction estimation unit 705 analyzes the visual line of the driver, the movement and the rotation of the head thereof, and the movement and the rotation of the upper body thereof on the basis of the states of the head and the upper body of the driver detected by the state detection unit 701 and the analysis result of the saccade of each of the right and left eyes of the driver by the single eye action analysis unit 704 to estimate the direction of the visual line movement of the driver.

An obstacle information acquisition unit 706 acquires information such as the direction of an obstacle existing around the vehicle and a distance to the vehicle on the basis of information from a sensor like a millimeter wave radar or a LIDAR (Light Detection and Ranging, Laser Imaging Detection and Ranging) and an in-vehicle camera such as, a stereo camera and a single lens camera installed in, for example, the vehicle. In other words, the obstacle mentioned here can be a target that should be recognized by the driver at a high consciousness level when the vehicle is switched from partially or fully automated driving to manual driving.

A pseudo target projection unit 707 projects and displays a pseudo target on, for example, a head up display in the visual point direction of the driver.

A saliency map generation unit 708 generates a saliency map that represents the probability distribution of positions naturally seen by the driver due to the visual characteristics of a target. Specifically, the saliency map generation unit 708 forms the saliency map by which the visual line of the driver can be turned on the basis of the information of an obstacle around the vehicle acquired by the obstacle information acquisition unit 706. In addition, the saliency map generation unit 708 adds, where necessary, a pseudo target displayed by the pseudo target photographing unit 707 to an obstacle existing in a real space in front of the vehicle where necessary to generate the saliency map.

An awakening degree determination unit 709 detects the correlation between the direction of the visual line of the driver estimated by the direction estimation unit 705 and the saliency map generated by the saliency map generation unit 708 to determine a consciousness level at which the driver recognizes an obstacle, i.e., the awakening degree of the driver with respect to the obstacle.

Processing performed by the awakening degree determination unit 709 will be supplementally described. The awakening degree determination unit 709 determines how often, how quickly, and how exactly the driver observes each event occurring in a real space in front of the vehicle during the traveling of the vehicle, and evaluates the presence or absence of a change in action characteristics or the slowdown degree of reaction based on recognition habit unique to the driver and a detection timing.

There could be a case that the driver is less likely to focus on the visual line when a landscape in front of the vehicle is really monotonous. In view of this, in order to stably use the awakening degree determination unit 709 at all times, it is effective that the pseudo target photographing unit 707 appropriately displays an image of a dummy virtual obstacle, i.e., a pseudo target on a head up display or the like in the visual point direction of the driver, and that the awakening degree determination unit 709 monitors whether the driver exactly responds to the pseudo target.

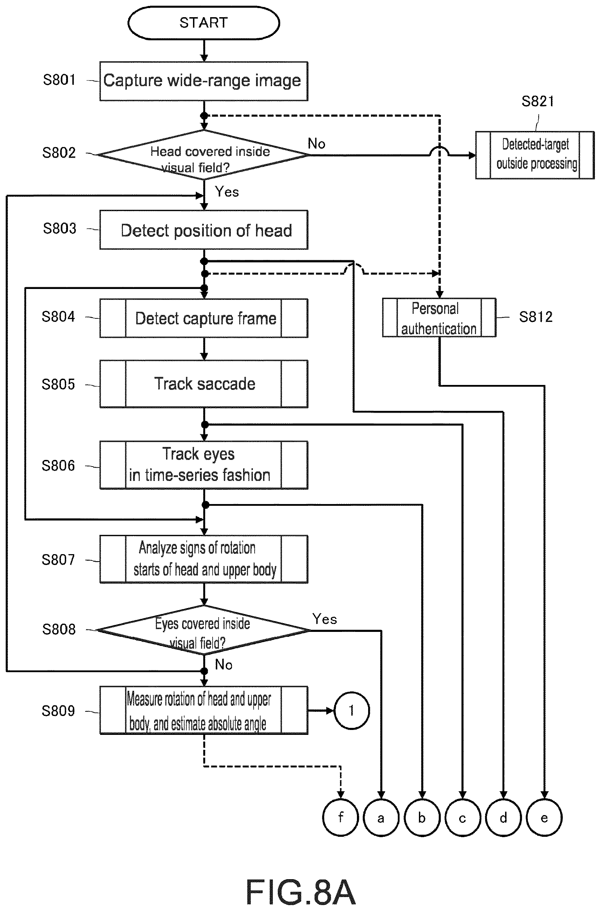

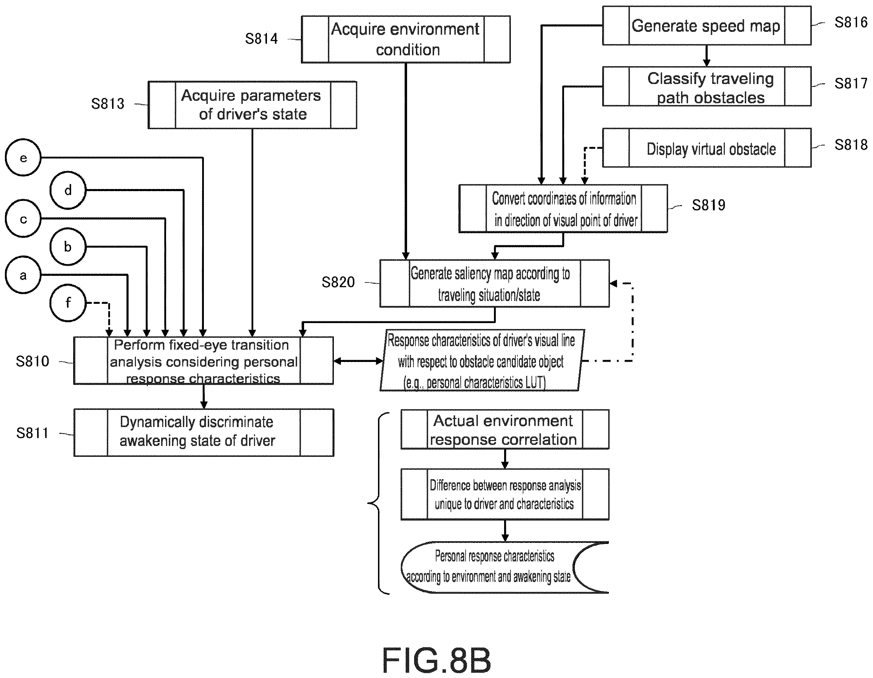

Figs. 8A and 8B show, in the form of a flowchart, a processing procedure for tracking the eyes of a driver and recognizing the consciousness level of the driver using the image processing device 700.

First, a wide-range image picked up at low speed of, for example, 60 fps or so and at low resolution is captured by an in-vehicle camera such as a driver monitor camera (step S801).

Next, a determination is made as to whether the position of the head of the driver is covered inside the captured wide-range image (or the visual field of the in-vehicle camera) (step S802).

Here, when the position of the head of the driver is not covered inside the wide-range image (No in step S802), detected-target outside processing may be performed (step S821).

On the other hand, when the position of the head of the driver is covered inside the wide-range image (Yes in step S802), the position of the head of the driver is detected from the whole region of the image picked up by the in-vehicle camera (step S803). Next, a range including eyes is estimated on the basis of the detected position of the head to detect the coordinates of a capture frame for tracking the eyes (step S804). The image data of the capture frame is read at a high frame rate of 1000 fps or so to track the saccade of the eyes of the driver in parallel (step S805). In addition, the attention destination of the driver is predicted, and a matching degree or a matching ratio with respect to the prediction is cumulatively analyzed in detail to estimate the movement destination of the eyes and track the eyes in a time-series fashion (step S806).

Then, on the basis of the detection result of the position of the head of the driver in step S803 and the analysis result of the saccade of the eyes in step S806, the signs of the rotation starts of the head and the upper body of the driver are analyzed (step S807).

The analysis of the signs in step S807 is performed to recognize sign movement in which the relative positions of the eyes are deviated from the capture frame by the movement of the head or the like and appropriately update the reset reading address of the capture frame. By appropriately analyzing movement estimation from the saccade of the eyes moving at high speed and setting the capture frame at the movement destination, an eye tracking loss is prevented.

Next, the rotation start of the upper body of the driver is estimated from the saccade analysis to determine whether the upper body rotates in a range coverable by the rotation of the eyes (step S808).

When the upper body does not rotate in the range not coverable by the rotation of the eyes (No in step S808), visual field information necessary for steering the vehicle cannot be obtained only with the saccade of the eyes. Therefore, the driver shifts to the rotation of the head and the upper body to continuously track the eyes with the supplement of the movement of the head.

On the other hand, when the upper body rotates in the range that can be covered with the rotation of the eyes (Yes in step S808), a fixed-eye transition analysis considering the personal response characteristics of the driver is performed (step S810).

In addition, when the upper body does not rotate in the range not coverable by the rotation of the eyes (No in step S808), the visual field information necessary for steering the vehicle cannot be obtained only with the saccade of the eyes. Therefore, the rotation of the head and the upper body is measured, and an absolute angle associated with the rotation is estimated (step S809). Thus, the fixed-eye transition analysis considering the personal response characteristics of the driver is performed (step S810).

In the fixed-eye transition analysis considering the personal response characteristics of the driver (step S810), the correlation between the estimated movement direction of the visual line of the driver and a saliency map is basically detected to determine fixed-eye transition, i.e., the awakening degree of the driver with respect to an obstacle. For example, when the eyes turn in conjunction with the direction of the saliency map in a real world space as its searching action of the saccade, the driver is highly conscious of driving.

Here, in the fixed-eye transition analysis considering the personal response characteristics of the driver (step S810), the driver is personally authenticated (step S812) in consideration of the fact that there is a large difference between individuals in visual line behavior characteristics to perform fixed-eye transition analysis processing based on the specified personal visual line behavior characteristics.

In addition, in the fixed-eye transition analysis considering the personal response characteristics of the driver (step S810), respective parameters indicating the state of the driver detected by various sensors installed in the vehicle are referred (step S813) in consideration of the fact that the visual line behavior characteristics of the driver change according to the state of the driver to perform the fixed-eye transition analysis processing.

In addition, a saliency map for use in performing the fixed-eye transition analysis of the driver is generated according to environment conditions under which the vehicle travels or the information of obstacles (step S820). The saliency map represents the probability distribution of positions naturally seen by the driver due to the visual characteristics of a target.

In step S820, the saliency map that can turn the visual line of the driver is formed on the basis of the information of obstacles around the vehicle. Therefore, in step S816, the directions and the relative distances of the obstacles in front of the vehicle are detected on the basis of information from a sensor such as a millimeter wave radar and a LIDAR and an in-vehicle camera such as a stereo camera and a single lens camera to generate a speed map. In step S817, targets to which the driver is assumed to pay more attention, i.e., targets (obstacle candidates) that exist on a traveling path or a non-road surface near the traveling path and are predicted to interfere with a self-vehicle traveling path in a near future among targets detected in step S816 are classified and sorted out with the assumption of the time-series transition of the traveling of the self-vehicle. In addition, in step S818, in order to more effectively monitor the awakening degree of the driver, an image of a dummy virtual obstacle, i.e., a pseudo target is appropriately displayed on a head up display or the like in the visual point direction of the driver. In step S819, the coordinates of the detected actual obstacle and the pseudo target displayed on the head up display are converted in the direction of the visual point when seen from the driver. Then, in step S820, the saliency map that can turn the visual line of the driver and corresponds to a traveling situation and state is formed on the basis of the information of the obstacle or the pseudo targets of which the coordinates have been converted. The details of a method for generating the saliency map will be described later.

In addition, in step S814, environmental conditions such as time zones, weathers, sunshine conditions, and road surface states under which the vehicle travels are acquired. In step S820, the saliency map is generated in consideration of such environmental conditions.

In addition, in step S810, visual line response characteristics for each driver with respect to an object as a candidate for an obstacle are referred to by a personal characteristics lookup table or the like to perform the fixed-eye transition analysis considering the personal response characteristics of the driver.

As for the eye movement of a driver, there is a difference between individuals in the visual line response characteristics with respect to an obstacle or an object as a candidate for the obstacle. Thus, in the present embodiment, personal characteristics on the eye movement of the driver such as the visual line response characteristics are managed in the personal characteristic lookup table, and the personal characteristics of the driver are sequentially learned on the basis of an analysis result in step S810.

Finally, in step S811, a consciousness level at which the driver recognizes the obstacle, i.e., the awakening state of the driver with respect to the obstacle is dynamically determined on the basis of the result of the correlation between the movement direction of the visual line of the driver and the saliency map.

The consciousness level of the driver recognized in step S811 is used to determine whether the switching of a driving mode is allowed or restricted or prohibited when the driving mode of the vehicle is switched from partially or fully automated driving to manual driving. When the consciousness level at which the driver recognizes the obstacle is substantially high, the switching from the automated driving to manual driving is allowed. On the other hand, when the consciousness level at which the driver recognizes the obstacle is low, the driver is not capable of responding to the manual driving and is risky. Therefore, the switching to the manual driving is restricted or prohibited. In addition, when the consciousness level of the driver is low, an alert such as producing an alert sound and applying a tactile or electric stimulus to the driver inside the vehicle during the switching of a driving mode or a manual driving period may be issued. Note that in order to cope with emergency, driver intervention control for the automated driving may be performed in which the vehicle is decelerated, slowed down, or stopped in stages according to circumstances under special conditions even if the driver partly has a dim consciousness.

Some of the processing defined in the flowcharts of Figs. 8A and 8B will be supplementally described below.

In the processing for setting the coordinates of the capture frame for tracking the eyes in step S804, the position of a range including the eyes predicted when the position of the head can be detected from the whole region of the pickup image is first estimated immediately after an operation starts. Then, the coordinates of the capture frame for tracking the eyes at a high frame rate are set according to the estimated positions of the eyes. During a driving action, the tracking information of the movement of the head or the eyes of the driver is fed back to predict the movement of the eyes and dynamically variably move the capture frame to prevent the eyes from being deviated from the capture frame within the range of the visual field of the in-vehicle camera. Note that when a plurality of camera systems are installed in the vehicle and the positions of the eyes of the driver move within a range across the visual fields of the adjacent camera systems, the tracking of the driver may be taken over between the adjacent camera systems.

In the tracking of the saccade of the eyes in step S805, it is assumed to use a laminated image sensor as shown in Fig. 4 or 5. Thus, it is possible to selectively transmit narrow-range and necessarily-toned partial image data of the capture frame to a signal processing IC laminated on the lower layer of a pixel region to perform tracking at high speed. The capture frame of the pixel region and the signal processing IC laminated on the lower layer are directly linked to each other and do not require a wiring loop therebetween. Therefore, a factor such as the impedance mismatch or the parasitic capacitance of a transmission path can be minimized, and the influence of electromagnetic radiation in proportion to load driving can be reduced with a reduction in a load. In addition, consumption energy can also be reduced with a reduction in the capacity of a driven target. Therefore, EMI (Electro Magnetic Interference) or EMC (Electro Magnetic Compatibility) characteristics can be remarkably improved.

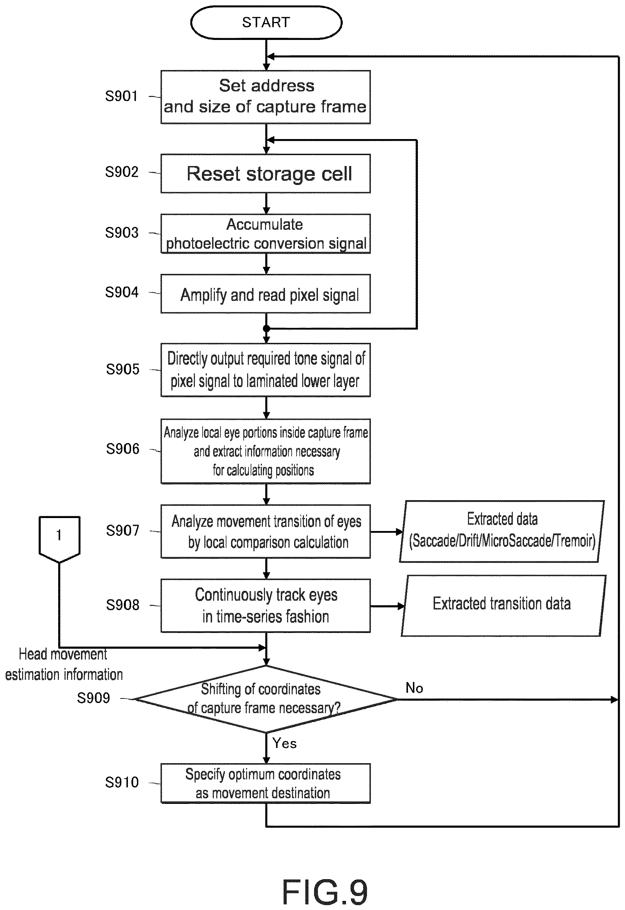

Fig. 9 shows, in the form of a flowchart, a processing procedure for tracking the saccade of the eyes at a high frame rate from the capture frame constituting a part of a pixel region of an image sensor in step S805.

First, the address and the size of a capture frame that reads a specific address at a high frame rate from the whole pixel region of an image sensor are set (step S901).

Then, the respective processing steps of resetting a storage cell (step S902), accumulating a photoelectric conversion signal (step S903), and amplifying and reading a pixel signal (step S904) are repeatedly performed in the pixel region.

Next, the pixel signal read from the pixel region is transmitted to a signal circuit region laminated on the lower layer of the pixel region (step S905). On this occasion, only a required tone signal of the pixel signal is transmitted. In addition, it is to be substantially understood that the pixel signal of the capture frame of the pixel region is directly output to the signal circuit region via the interface wiring illustrated in Fig. 3 to make its noise reduced.

The processing of the signal circuit region will be described below.

First, information necessary for analyzing local eye portions and calculating the positions of the local eye portions inside the capture frame such as the contours of the eyes and the reflection center of reference light is extracted (step S906).

Next, the movement transitions of the eyes are analyzed at a low data rate by local comparison calculation using a memory region (step S907). Here, the memory region is assumed to be laminated on the signal circuit region (see, for example, Fig. 5) or is assumed to be arranged in the same layer as the signal circuit region. In step S907, data on peculiar eye movement such as saccade, drift, microsaccade, and tremor is extracted.

Then, the time-series continuous rotations of the eyes, i.e., the saccade of the eyes is tracked (step S908).

Here, head movement estimation information obtained in step S809 is acquired to determine whether it is necessary to shift the coordinates of the capture frame set in step S901 (step S909).

When it is not necessary to shift the coordinates of the capture frame (No in step S909), the processing returns directly to step S901 without correcting the coordinates of the capture frame to repeatedly perform the same processing described above. In addition, when it is necessary to shift the coordinates of the capture frame (Yes in step S909), the processing returns to step S901 after specifying optimum coordinates as the movement destination of the capture frame (step S910) to repeatedly perform the same processing described above.

The processing of step S809 will be supplementally described. By the analysis of the acceleration action of eyes, the necessity of rotating the head or the upper body to compensate for the eyes can be predicted. In addition, when such rotation occurs, the eyes rotate to cancel out a relative rotational angle with respect to the head. Therefore, it is necessary to analyze a visual line in consideration of the fact. In addition, when trying to see a certain target in a state in which a consciousness level is low, it is assumed that the eyes should confirm the target psychologically. Therefore, in order to make, for example, the head or the upper body eagerly move in a state in which an awakening level is low, the eyes extraordinarily follow the action as their characteristics. Therefore, the analysis of the characteristics of the temporal behavior of the head or the upper body in conjunction with the analysis of the visual line of the eyes gives the driver a clue for determining the consciousness level. By complementarily using the movement ranges and the acceleration characteristics of the eyes, it becomes possible to more exactly determine the state of the driver.

The processing of step S810 will be supplementally described. During the traveling of a vehicle, a driver's visual line attention with respect to a surrounding environment is determined by a plurality of complicatedly tangling factors. That is, fixed-eye transition cannot be analyzed only with visual information as the light energy of a simple physical optics scene of a forward scene during traveling.

However, the fixed-eye transition analysis is roughly constituted by the step of selectively fixing eyes on and interpreting, when traveling on a traveling setting route meeting a driving purpose, a visual image catching light energy entering from the front side of the traveling on the basis of a visual sense, an auditory sense, and an intellectual attention. The first visual line movement (by saccade or the movement of a head) is caused as a feedback in response to a trigger for the movement of a visual line after receiving an induced factor. The first visual line movement is not necessarily performed only with the reception of an optical stimulus from a target not related to the traveling or a target to which attention is not required to be paid.

Information captured as light energy is received by receptor cells as a signal, and the signal is taken over to bipolar cells and transmitted to the brain of a visual area via ganglion cells associated with thinking. Information received by the driver at an initial stage is generally vague information spreading in a surrounding visual field. Then, a visual line is moved in the highly salient direction of a target having a large temporal light intensity differential, whereby a target can be captured at a narrow central fovea or a parafovea in a state of facing an approximate target. Above all, due to a detailed image of a target obtained at the central fovea or the parafovea or a delay in adjusting the focal points of eyes until the acquisition of the detailed image, perception and recognition in the following stages are also delayed according to situations.

When the ability to adjust the focal points of the eyes is temporarily reduced due to the fatigue of the eyes or any physical condition fluctuating factor, a stop time during the fixation of the eyes becomes long. Particularly, unless information with high priority suddenly appears in a state in which new visual information sequentially enters a surrounding visual field, the stop time becomes long. In addition, when the content of a visually-recognized target is figured out (or its interpretation is completed) from experimental knowledge even if the focal points of the eyes are not focused, the stop time of the eyes for visual fixation becomes long. In this case, the visual line is not fixed, but saccade for a target with the next-highest priority that is sequentially input starts. For example, a road boundary line such as a white line is important information for stipulating traveling conditions, but it is not necessary to fix the visual line to the road boundary line to see the same. Drivers accustomed to driving do not fix their eyes to strict white line positions during normal traveling.

On the other hand, in a situation in which the focusing ability of the driver is degraded due to the fatigue of the eyes, a reduction in eyesight, or the like and focusing is not adjusted even if any longer time is taken when an eye stop period during visual fixation increases with a reduction in the focal-point function of the eyes, the driver would rather move a visual line to search for a neighboring place at an early period according to a situation grasping emergency and increase a confirmation degree with a surrounding vague image. The shifting frequency, the shifting range, and the like from the stop of the visual fixation to microsaccade or the like are experimental characteristics unique to the driver. That is, the behavior of the comprehensive eye movement of the visual fixation or the saccade of the driver is separately analyzed for each authenticated driver to suit a target operation, whereby a state unique to the driver is accurately grasped.

Depending on movement characteristics, eye movement called fixational eye movement is classified into the respective states of microsaccade in which eyes move relatively large and quick, drift in which the eyes move large and slow, and tremor in which the eyes move small and at a high frequency. The states change according to environment conditions (fine weather and fine visibility, rainy weather, nighttime, blizzard, glaring oncoming vehicle, ...), physiological and physical visual states (such as a state in which the eyes are finely focused and a situation in which it takes time to focus the eyes due to fatigues or the like), and consciousness states unique to a driver (elements reflecting consciousness states in a brain directly linked to the transmission of neuron signals such as element interpretation and memory reference performed in the order of a primary visual area, a visual association area, and a high-order association area). In addition, in the high-order association area, priority is placed on searching for an important element related to an objective trip rather than referring to the storage of a target without a condition in the movement of a visual line to discriminate the purpose of steering for driving. Therefore, in parallel with the analysis of the movement of the visual line, a list of visual-line movement destination targets according to trips is issued to the driver.

These high-order factors are complicatedly correlated with each other according to situations to result in the movement of the visual line of the driver, and their mutual influences or weightings depend on experimental characteristics unique to the driver. Therefore, as for the discrimination of consciousness states unique to the driver, it is preferable to accumulate and learn these characteristics in a stereotyped fashion to a certain degree and uses the same as indexes for discriminating the states of the driver when the same vehicle travels or when traveling under a similar environment. When the visual line characteristics of the driver are observed from a statistical standpoint, the consciousness states of the driver have certain tendencies corresponding to a driving environment. For example, it is assumed that the response characteristics of a braking device in such a state are accelerated to prompt the driver to have a rest or improve the braking start characteristics when fatigues tend to increase. However, it is difficult to discriminate the actual detailed consciousness level unique to the driver according to a determination based on a threshold set from the statistical tendency. Consequently, the determination is not suitable for determining whether driving steering should be taken over from automated driving to manual driving. In order to more exactly grasp states unique to the driver, it is necessary to make a determination based on the characteristics unique to the driver. In addition, discrimination conditions also change according to a driving environment or situation. That is, variable values with which the consciousness states of the driver are determined are multi-dimensional, and correlated variable conditions cannot be necessarily expressed as independent orthogonal coordinate variables. The connection between complicated and different correlated parameters such as an accelerator pedal operation, a braking operation, a steering operation, a seating attitude, a visual confirmation range, each road surface state dependence, weather dependence, self-vehicle loaded situation dependence, a traveling road state, application dependence, and a breathing state change with the habit or the like of the driver. Here, a learning method with artificial intelligence such as deep learning that has been actively applied in recent years is a method effective for discriminating the personal states of the driver having abundant personal characteristics since a learning device itself repeatedly performs self-selection weighting on a correlated dimension on the basis of multi-dimensional information to recognize the same. In addition, the method can be a learning method adapted to actively and momentarily changing drivers or environmental changes.

Then, as a driver's state confirmation sequence, it is effective to confirm, for example, the feedback active reaction of a final determination.

Here, as an example of the feedback active reaction corresponding to the state of the driver, the soft operation of a brake pedal requiring a variable and intellectual determination reaction on a head up display is assumed to be achieved by the combination of a reaction response button operation and a specific gesture operation, for example, when a reduction in the determination ability of the driver is assumed. As an example, a dummy road sign is displayed on a head up display, and a numerical-value reaction or the like on the road sign is visually recognized.

As for a driver's visual line attention with respect to a surrounding traveling environment, an attention level in a visual line direction actively changes due to the fatigue, the mental state, the experience, or the like of the driver on a case-by-case basis. However, its wide distribution shows a moderate change with time, and similar distribution characteristics are expected so long as the driver has substantially the same traveling environment, physical condition, and mental state. This attention level distribution curve is classified into a plurality of stages of levels, and the level of a central eyesight is classified to compare the surrounding attention situation of the driver currently in a traveling state with a saliency map unique to the driver in terms of the correlation of time-series visual-line movement destination characteristics, whereby it becomes possible to grasp the status of the attention level of the driver.

There is a rapid change point when the driver is overcome by primary drowsiness. However, it is also possible to see a sign in step S807 or the like. Accordingly, the fixed-eye transition analysis in step S810 is effective for determining the awakening restoration degree of the driver, for example, when shifting from partially or fully automated driving to manual driving.

Above all, it is indispensable to transmit a signal to a thinking neural circuit to determine a situation when the driver continues to drive on the basis of his/her intellectual determination. The analysis of elements reflecting a consciousness state in the brain directly linked to the transmission of a neuron signal such as element interpretation or memory reference performed in the order of a primary visual area, a visual area association, and a high-order association area is largely influenced on the interpretation of the situation of a target to which the driver turns his/her eyes. Generally, a target is grasped in a central visual field. Therefore, the awakening state of the driver is largely reflected by dynamic behavior during visual fixation. Conventionally, some products that respond to the characteristic minute movement of the driver to analyze the state of the driver have been manufactured. However, an unwearable device with which the driver cumulatively analyzes his/her behavior over a long period without consciousness has not been presented. On the other hand, according to the technology disclosed in the present specification, the high speed and minute behavior of the eyes of the driver moving widely in a free style state can be captured, and the minute behavior of the eyes and a driving situation or environment correlation are grasped by machine learning as characteristics unique to the driver so that the behavior of the eyes and the characteristics can be compared with each other. Thus, it becomes possible to accurately grasp the awakening state of the driver even under a driving environment that changes momentarily.

In summary, in step S810, it becomes possible to analyze characteristics unique to the driver such as a visual-fixation stop period, an occurrence amount of microsaccade, and a search range analysis in detail and estimate the state of the driver from cumulative learning characteristics unique to the driver and the sequential most-recent visual line analysis of the driver.

Aside from the attention level distribution of a visual area with respect to a central visual field, surrounding visual line movement is adequately repeated to reduce a perception loss to consciously or unconsciously compensate for the narrowing of the attention level distribution of eyes only. When the attention level of a surrounding visual field reduces with age or fatigue, it is effective to positively move a visual line to reduce the entangling accident of a pedestrian at the time of, for example, turning right and left. From the correlation between a visual-fixation stop period and the frequency with which the eyes perform saccade in the direction of a surrounding visual filed before moving the visual line so as to follow a highly-salient target in a saliency map, a determination can be made as to whether the saccade compensates for a reduction in the attention level of the surrounding visual field.

In addition, the driver turns to a target to which attention should be paid and fixes his/her visual line to understand actual details and advance the determination of a sensory area in a brain in so-called the stop period (for example, the shadow of a pedestrian is caught in the surrounding visual filed, the eyes rotate and turn in the saccade, and the eyes are focused in the process of visual fixation to specifically determine whether the pedestrian enters or leaves the path of a self-vehicle from the observed face, the attitude, or the like of the pedestrian). When obtaining the positive proof of a situation to a certain degree via a visual area, the driver shifts to a sweep action to search for other attention targets to dimly recognize a visual line in a surrounding visual filed. When there is any target that should be confirmed preferentially, the driver appropriately moves the visual line in a highly-salient direction in a saliency map to advance the confirmation of a situation. Such a distribution of targets that should be sequentially confirmed is exactly the saliency map used in the fixed-eye transition analysis of the driver in the present embodiment.

That is, a map in the visual line direction of the driver showing what target the driver is likely to see with a high probability is used in the present embodiment. In step S820, it is effective to use a map in which the occurrence probability of an obstacle on the path of a self-vehicle important for a driving operation is weighted in the general-purpose saliency map of a pure driver's visual-line image. The details of step S820 will be described later.

The visual line movement of the driver, i.e., a determination as to whether the driver moves the visual line to a next target after completing the determination of a situation is influenced by the saliency of an outer world seen from the visual line of the driver.

It is necessary for the driver to simultaneously and parallelly determine the direction of the path of the traveling vehicle and a target including its surrounding road surface. In various environmental scenes appearing in a combined fashion, the driver appropriately and sequentially overlooks the direction of the path of the vehicle and its surrounding according to an importance degree and a recognition and comprehension degree to continue a traveling operation. The driver performs peculiar eye movement such as saccade, microsaccade, drift, and tremor according to a traveling situation such as an environment condition and an own state (a physiological and physical visual state, a consciousness state unique to the driver).

In a visual fixation stop period and the period of microsaccade, target dependency to recognize a target is accompanied. Therefore, by finding a correlation with the classification of a target having high saliency on the saliency map, it becomes possible to more exactly determine the state of the driver (awakening level). On this occasion, a recognition distance or an external environment also becomes a fluctuation factor. For example, the driver just looks askew at a highway exit sign when he/she is not scheduled to use the exit. However, as for a right/left turn prohibition sign or a time zone limit road sign, it takes time since the user is required to complete the recognition and interpretation of a time zone. In addition, the driver does not necessarily perform recognition and interpretation to confirm right and left sides when he/she is on a priority road at an intersection. Otherwise, the driver confirms the right and left sides in a certain range. In addition, when seeing the green arrow of a distant traffic signal or the like, the driver is likely to fix the eyes for a longer time compared with a case in which he/she sees a simple red signal. However, any driver with really fine eyesight does not necessarily visually fix the eyes, and a difference between both cases is not significant.

The processing of step S811 will be supplementally described. The saliency map is generated by weighting an attention target to which the visual line is turned according to an environment condition (such as an urban area, a highway, a nighttime, rainy weather, and blizzard). In addition, when the environment condition changes halfway, weighting characteristics are updated. In an urban area, particularly when any vehicle breaks in from right and left sides in heavy traffic, when paying attention to a weaving motorcycle, or when traveling in a school zone, the driver moves the visual line to widely confirm right and left sides. In an environment in which a pedestrian is not assumed to run out due to an ordinary dedicated street crossing in an urban area, the driver turns the visual line to a short or middle distance forward side, a traffic signal, or the like with a high probability. In addition, when the driver plans to travel on a route in which a road is not branched to a dedicated street road or where no vehicle enters, the driver turns the visual line mainly to a middle distance side. In addition, when traveling in an environment in which a visual field is poor due to bad weather or when traveling in rainy weather, the driver is highly likely to focus attention to a forward side. Therefore, the driver is less likely to move the visual line to a surrounding but is highly likely to search for a middle distance range with the visual line. However, even in the same environment, the driver may respond to a specific situation more sensitively and frequently perform visual fixation on an attention target on the basis of, for example, the incident or the accidental experience of the same driver in the past. In addition, even in the same rainy weather, an occupational driver such as a taxi driver performs an operation to intermittently confirm the behavior of a shoulder pedestrian particularly at its free time. Thus, the characteristic behavior of the driver is different depending on the need of the driver even in the same environment condition. For each of a plurality of driving trips, characteristics unique to the driver are accumulated and learned. By making a comparison using the learned characteristics and a look up table or the like, it becomes possible to overlook the transition of the current state of the driver.

The personal authentication performed in step S812 will be supplementally described. The visual line behavior characteristics of the driver are largely different between individuals. Therefore, in order to perform the fixed-eye transition analysis of the driver in step S810, it is necessary to specify the driver as a solid target and consider the personal response characteristics. The personal authentication is a desirable system for specifying a solid target. However, living-body authentication is not necessarily performed by the acquisition of living-body information of the face, the eyes, or the iris of the driver, and the living-body information may be acquired by a specific personal code, fingerprint authentication, voice authentication, vein authentication, or the like. Thus, the personal authentication is not limited to a specific authentication method.

The method for acquiring the state of the driver performed in step S813 will be supplementally described. At the filing of the present application, many sensors or information analysis methods applicable to determine the state of the driver have been proposed. The state of the driver can be acquired using, for example, the operating way, the operating frequency, the pressing degree of a brake pedal or an accelerator pedal by the driver, the operating stability of a steering wheel, a contact type or non-contact type heart rate analysis, a mental state analysis based on a heart rate, an alcohol intake amount analyzer, a fatigue analysis based on a body odor analysis, a brain wave analysis, an electrocardiogram, a body temperature or a body temperature distribution, an attitude analysis during driving, a breathing analysis, an active response reaction with respect to a sound wave or audio and haptics vibration, or the like. By combining two or more of the above methods together to interpolate the state analysis value of the driver, it also becomes possible to recognize the state of the driver with high accuracy.

The environment condition acquired in step S814 will be supplementally described. When traveling on a maintained vehicle-dedicated lane in the daytime, the driver is capable of traveling (operation steering) in a relatively relaxed state. On the other hand, the driver pays much attention to a surrounding environment depending on an environment factor such as traveling in the nighttime in which a street lamp is dark and in an environment in which an unintended obstacle such as a man and an animal may enter in a direction other than the direction of a forward visual field or in rainy weather, fog, and snow by which a visual field is disturbed, traveling against backlight, traveling on a risky road due to its frosted surface, on a bridge, in a tunnel, in a traveling environment in which sidewind is strong, on a road on which a road sign cautioning about a wild animal is installed, and on an unfamiliar road, and traveling at a point at which accidents frequently occur. Therefore, in consideration of the above environment factors such as the frequency of the confirmation of a surrounding environment and the range of the movement of the visual line for the confirmation, the dynamic state of the driver can be more accurately analyzed.

The processing performed in step S816 will be supplementally described. In step S816, information acquirable by various surrounding environment sensing with a sensor such as a millimeter wave radar and a LIDAR and an in-vehicle camera such as a stereo camera and a single lens camera is acquired, and the labeling of a target such as a detected obstacle and its position or the like are converted into a direction, a distance, and (a relative distance, relative speed, and relative acceleration) in an integrated fashion to perform map generation processing. Then, in step S819, the coordinates of the information are converted in the visual point of the driver to dynamically generate data indicating the direction or the speed of each detected obstacle for the driver.

The processing for removing an obstacle on a traveling path performed in step S817 will be supplementally described. From bird's-eye data in which a road surface in a traveling direction is projected, a target that exists on a route on which the vehicle is scheduled to move and that is highly likely to hinder the traveling of the self-vehicle when the vehicle continues to move forward is removed. That is, a target to which the driver is assumed to pay more attention among obstacles detected in front of the vehicle is removed as a target to which the driver frequently moves the visual line or frequently fixes the visual line.

The processing for displaying a pseudo target performed in step S818 will be supplementally described. If a traveling road and its surrounding environment are too monotonous when a determination is made as to whether the driver recognizes a state on the basis of the saccade of the eyes, the frequency with which the correlation between the visual line of the driver and an actual environment can be monitored reduces, which results in a difficulty in examining the reaction of the driver with high accuracy at an appropriate timing. Therefore, a pseudo target composed of a virtual image is displayed to examine whether the driver can exactly respond to a plurality of pseudo targets with appropriate response characteristics. For example, a pseudo target is displayed so as to be seen by the driver using a non-contact type visual input device such as a head up display. For the purpose of discriminating the consciousness state of the driver, a pseudo target may be displayed with the characterization of not only a direction but also a determination response feedback such as, for example, a color and a numerical value including conscious perception activation confirmation. The response mode of the driver is basically a case example assumed by analyzing the behavior of the saccade or the like of the visual line. Nevertheless, the advantage of using the visual line is that the consciousness state can be discriminated in a state in which the driver behaves unconsciously, seamlessly, and naturally.

The processing for generating the saliency map according to a traveling situation performed in step S820 will be supplementally described. The observation of a surrounding environment by the driver during traveling largely changes depending on a traveling situation. For example, in a situation in which the driver is not required to pay much attention to a forward side since a distance between vehicles is substantially ensured on an empty highway, a situation in which the driver does not predict when a pedestrian runs out in an urban area in the nighttime, a situation such as an environment and a school zone in which a road surface is slippery due to snow or frost, or the like, the driver is forced to drive while confirming a surrounding area on the basis of the unique characteristics of the visual movement according to an experience, habituation, or the like. The saliency map corresponding to the traveling situation of the vehicle or the state of the driver shows characteristics unique to the driver, stores the information of the traveling history of the driver, and is structured through learning. In addition, the saliency map shows characteristics changing depending on an environment, a physical condition, or the like. Therefore, the saliency map classifies and stores the traveling history of the driver according to an environment situation. For each similar traveling environment, the driver refers to the saliency map and analyzes the dynamic response characteristics of the visual line with respect to the map in a time-series fashion to calculate back and analogize the attention level of the driver.

The detection target outside processing performed in step S821 is made different according to the purpose or the use situation of the image processing device 700. For example, when the processing is used in the time-series management of the ability to restore to the awakening state by the driver to dynamically monitor the awakening state of the driver of the vehicle in a fully automated driving state, it is assumed to shift to safe slow-down traveling, evacuation and stop, or a stop sequence if the ability to restore to the awakening state is determined to be insufficient. In addition, when monitoring the state transition of the driver is not directly linked to a critical determination as in monitoring the visual line of the user like, for example, a CMS, an alert sound or an alert may be appropriately presented. In addition, when the driver moves widely inside the vehicle in a free style state rather than seating on a driver's seat in a fixed attitude with the widespread of automated driving, it is assumed that the driver cannot be captured only with one camera system. Therefore, a use mode in which a plurality of camera systems are installed in the vehicle and the tracking of the driver is taken over between the adjacent camera systems is also assumed.

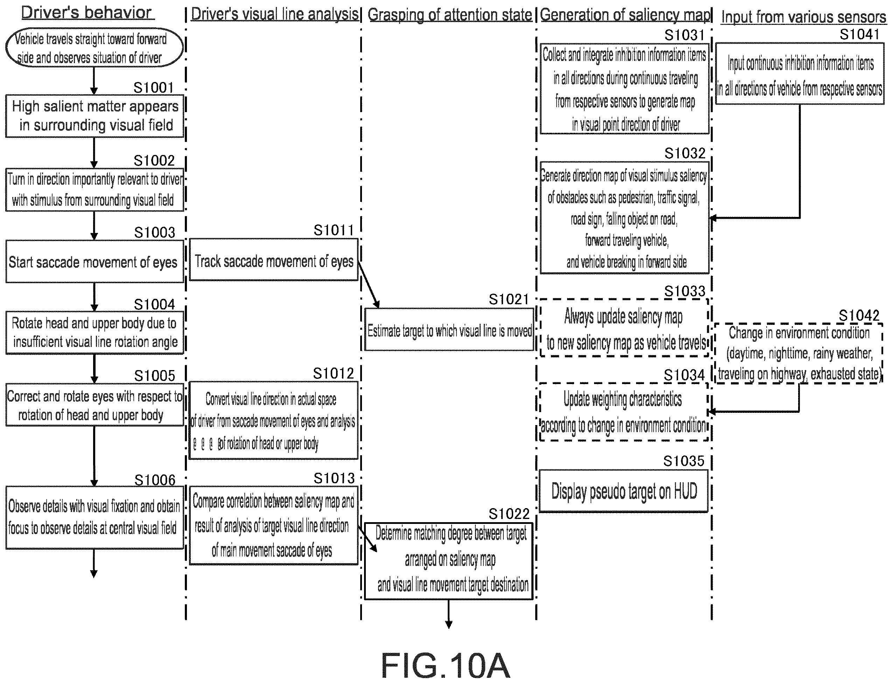

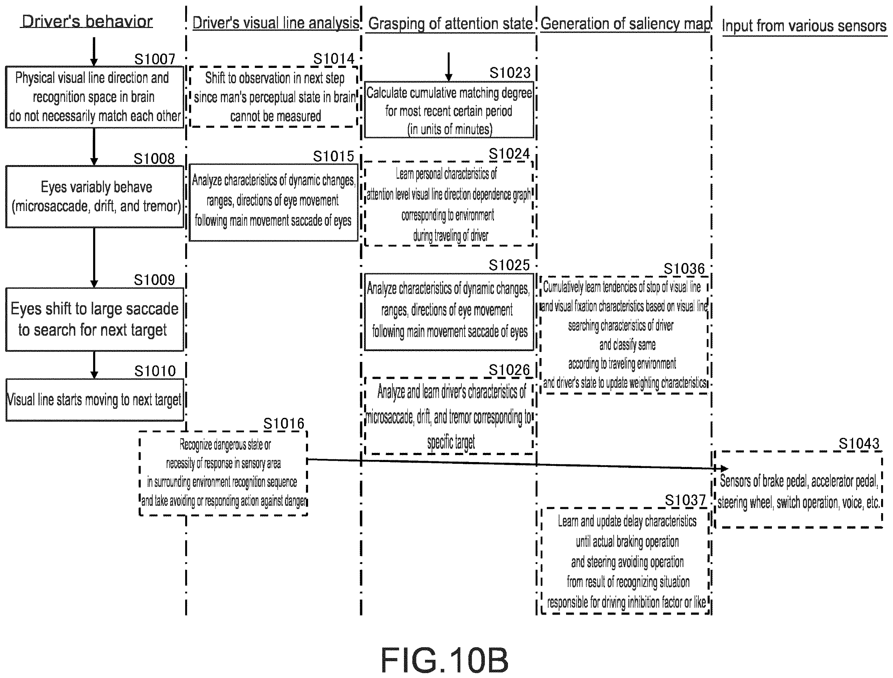

Figs. 10A and 10B summarize the respective processing of the analysis of the visual line of a driver, the grasping of an attention state (awakening level), the generation of a saliency map, and the inputs of various sensors (driver's state parameters) that are performed simultaneously with the behavior of the driver.

When a vehicle travels straight toward a forward side and observes the situation of the driver, a highly salient matter appears in the surrounding visual field of the driver (step S1001). Then, the driver detects a direction importantly relevant to the driver with a stimulus from the surrounding visual field (step S1002) and starts the saccade of eyes (step S1003) .

Here, when a visual line rotational angle with the wiggling rotation of the saccade of the eyes is insufficient and thus the driver cannot capture a target, the driver subsequently rotates the head and the upper body (step S1004). Note that the driver does not rely only on visual information to recognize a surrounding environment but uses other senses such as an auditory sense, vibration, odor, and the blinking of a flash light emitted from an emergency vehicle or the like as determination factors. Further, when recognizing an impact sound, a collision, a collision sound, moment flash light blinking, or the like from a backward side outside the surrounding visual field, there could be a case that the driver first changes his/her head or body attitude and then the eyes follow the head or the body attitude. Therefore, the saccade of the eyes does not necessarily serve as a trigger for the first turning movement.

Then, when rotating the head or the upper body, the driver corrects and rotates the eyes to maintain the visual line in an objective direction (step S1005). That is, the driver temporarily largely rotates the eyes with respect to the head to cancel excessive rotation with respect to the head. In the case of a head mount type visual line detection device, the canceling correction processing is required to analyze relative eye movement with respect to the head. However, in the detection of the driver in a free style state, the eye movement is measured on the basis of coordinate space fixed to the vehicle. Thus, the eyes are only required to be appropriately corrected in the detection according to a situation.

As the capturing of the target is completed as described above, the driver starts observing details with visual fixation and at the same time obtains focus to proceed to the observation of the details at the central visual field (step S1006).

When the target is actually observed in the operations of the visual fixation and the observation of the details in the above sequence, the physical visual line direction and recognition space in the brain do not necessarily match each other (step S1007).

Specifically, in the physical direction, the eyes perform variable behavior such as so-called microsaccade, drift, or tremor during the visual fixation.

Generally, when the driver does not aim to interpret one matter in detail as in reading a book, the eyes stop in a short period of time during the visual fixation. The eyes are not required to repeatedly generate microsaccade, drift, and tremor in a combined fashion but shift to big saccade for searching for a next target (step S1009), and start moving the visual line to the next target (step S1010).

As the eyes of the driver start the saccade (step S1003), the saccade of the eyes is tracked as the visual line analysis processing of the driver (step S1011). Then, on the basis of the tracking result of the saccade of the eyes, a target to which the visual line is moved is estimated as processing for grasping the attention state of the driver (step S1021).

In addition, as the driver corrects and rotates the eyes (step S1005), a visual line direction in the real space of the driver is converted from the saccade of the eyes and the analysis of the rotation of the head or the upper body as the visual line analysis processing of the driver (step S1012). Then, the correlation between the saliency map and the result of the analysis of the target visual line direction of the main movement saccade of the eyes is compared (step S1013). The physical visual line direction of the driver and recognition space in the brain do not necessarily completely match each other (step S1007). However, since a perception state in the brain of a man is not directly measurable, the visual line analysis processing of the driver actually shifts to observation in a next step (step S1014).

Then, as processing for grasping the attention state of the driver, the matching degree between a target arranged on the saliency map and a visual line movement target destination is determined by the measurement of a distance to the nearest maximum salient value or the like (step S1022), and a cumulative matching degree for a most recent certain period (in units of minutes) is calculated (step S1023). Here, the personal characteristics of an attention level visual line direction dependence graph corresponding to an environment during the traveling of the driver are learned (step S1024). Note that there has been reported a symptom in which the driver suddenly becomes drowsy even in an awakening state and suddenly becomes unconscious due to an illness called a sleep apnea syndrome in recent years. Therefore, it is also important to observe progress in which a consciousness state conclusively reduces in the observation of chronological transition. The frequency or the interval of the above periodic observation in a certain period is desirably performed according to the characteristics of the driver.

The eyes of the driver perform peculiar movement such as microsaccade, drift, and tremor during visual fixation (step S1008). However, as processing for analyzing the visual line of the driver, the characteristics of the dynamic changes, the ranges, the directions of the microsaccade, the drift, the tremor, or the like following the main movement saccade of the eyes are analyzed (step S1015).

In addition, as the processing for grasping the attention state of the driver, the characteristics of a correlation with the observation of a visual fixation target are analyzed, and a target to which the driver is assumed to pay more attention is classified (step S1025). Here, the driver's characteristics of microsaccade, drift, and tremor corresponding to a specific target are analyzed and learned as personal characteristics (step S1026).

As saliency map generation processing, inhibition information items in all directions of the vehicle during continuous traveling are collected from respective sensors installed in the vehicle and integrated with each other to generate a map in the visual point direction of the driver at a front stage (step S1031).

Then, continuous inhibition information items (directions and relative distances of obstacles) in all directions of the vehicle are input from respective sensors such as a millimeter wave radar and a LIDAR (step S1041). As the saliency map generation processing, the direction map of the visual stimulus saliency of obstacles such as a pedestrian, a traffic signal, a road sign, a falling object on a road, a forward traveling vehicle, and a vehicle breaking in a forward side is generated (step S1032). Then, the saliency map is always updated to a new saliency map as the vehicle travels (step S1033).

In addition, during the generation of the saliency map, distribution weighting is performed on the saliency map mechanically obtained by an image analysis according to a traveling situation or state. In addition, when an environment condition (daytime, nighttime, rainy weather, traveling on a highway, an exhausted state) changes halfway (step S1042), weighting characteristics unique to the driver according to the environment condition are updated (step S1034).

In addition, in the saliency map generation processing, an image of a dummy virtual obstacle, i.e., a pseudo target is appropriately displayed on a head up display or the like in the visual point direction of the driver in order to more effectively monitor the awakening degree of the driver, and visual line response characteristics with respect to the pseudo target are seen to be determined (step S1035). This is because if a traveling road and its surrounding environment are too monotonous, the frequency with which the correlation between the visual line of the driver and an actual environment can be monitored reduces, which results in a difficulty in examining the reaction of the driver with high accuracy at an appropriate timing. For the recognition of the state of the driver, the analysis of the visual line of the driver is mainly described above. However, the thinking determination response result of the driver may be used in combination to make a determination. For example, a method for detecting a thinking and determination result with respect to an audio guidance inside the vehicle as the voice response of the driver, a method for moving the visual line to a specific pattern displayed on a head up display along an audio analysis, and an active and seamless method in which a detection result is obtained via the thinking sequence of the driver such as the button pressing operation reaction of a specific pattern mocking a Morse code are effective and robust methods for discriminating the awakening state of the driver.

In addition, in the saliency map generation processing, the tendencies of the stop of the visual line and visual fixation characteristics are cumulatively learned on the basis of the visual line searching characteristics of the driver and classified according to a traveling environment and a driver's state to update weighting characteristics (step S1036).

In addition, in the saliency map generation processing, delay characteristics until an actual braking operation and a steering avoiding operation are learned and updated from the result of recognizing a situation responsible for a driving inhibition factor or the like (step S1037). Here, the situation recognition of the driving inhibition factor is actually the recognition of the presence or absence of the visual fixation or the stop of the visual line with respect to a target on the saliency map that could become an obstacle with a high probability.

When recognizing a dangerous state or the necessity of a response in a sensory area in a surrounding environment recognition sequence, the driver takes an avoiding or responding action against the danger (step S1016). Such an action can be detected by means of the operation of a pedal such as a brake pedal and an accelerator pedal, a steering wheel, and a switch, the detection of emitted voice and gesture, and other sensors (step S1043).

### Industrial Applicability

The technology disclosed in the present specification is described in detail above with reference to the specific embodiments. However, it is obvious that persons skilled in the art could correct or replace the embodiments without departing from the scope of the appended claims.

The technology disclosed in the present specification can be applied to various vehicles such as automobiles (including gasoline-powered vehicles and diesel-powered vehicles), electric automobiles, hybrid electric automobiles, motorcycles, bicycles, and personal mobilities and also to movable bodies other than vehicles traveling on roads to allow the acquisition of the awakening degree of drivers based on the tracking of the saccade of the eyes of the drivers. Of course, the use of the technology disclosed in the present specification is not limited to the drivers of movable bodies, but the technology can be similarly used to acquire the awakening degree of men performing various operations.

In short, the invention is carried out according to the appended claims.

### Reference Signs List

- 100: in-vehicle camera
- 201: image pickup element
- 202: image recognition processing IC
- 203: interface wiring
- 400: laminated image sensor
- 410: pixel region
- 420: signal processing circuit region
- 421: camera signal processing unit
- 422: face position tracking unit
- 423: eye region detection unit
- 424: high-speed reading unit
- 500: memory-installed laminated image sensor
- 510: pixel region
- 520: signal processing circuit region
- 530: memory region
- 521: camera signal processing unit
- 522: face position tracking unit
- 523: face image writing unit
- 524: selective high-speed reading unit
- 700: image processing device
- 701: state detection unit
- 702: area selection specification unit
- 703: eye saccade analysis processing unit
- 704: single eye action analysis unit
- 705: direction estimation unit
- 706: obstacle information acquisition unit
- 707: pseudo target projection unit
- 708: saliency map generation unit
- 709: awakening degree determination unit
- 701: whole region
- 712: selective high-speed reading area

## Claims

1. An image processing device, comprising:
a first image acquisition unit (421, 521) that is configured to acquire an image of a driver of a vehicle at a first frame rate;
a region determination unit (423, 702) that is configured to determine a region including eyes of the driver in the image acquired by the first image acquisition unit;
a second image acquisition unit (424, 524, 703) that is configured to acquire an image of the region at a second frame rate higher than the first frame rate;
a processing unit that is configured to process an image of the region acquired at the second frame rate to recognize or discriminate a consciousness level of the driver; and
a control unit that is configured to control switching of a driving mode of the vehicle according to the consciousness level of the driver recognized or discriminated by the processing unit,
wherein the processing unit is configured to recognize or discriminate the consciousness level of the driver according to a tracking result of eye movement of the driver on a basis of the image in the region,
wherein the processing unit is configured to track at least one action of saccade and microsaccade of the eyes of the driver, and
wherein the processing unit is configured to recognize or discriminate the consciousness level of the driver on a basis of a comparison result between the eye movement of the driver and a saliency map by detecting the correlation between the direction of the visual line of the driver and the saliency map, wherein the saliency map is a two-dimensional distribution map that represents the probability distribution of positions naturally seen by the driver due to the visual characteristics of a target.

2. The image processing device according to claim 1, wherein
the control unit restricts or prohibits switching from automated driving to manual driving of the vehicle when the consciousness level of the driver is a prescribed level or less.

3. The image processing device according to claim 1, further comprising
an alert unit that issues an alert when the consciousness level of the driver is a prescribed level or less.

4. The image processing device according to claim 1, wherein
the region determination unit corrects a position of the region according to rotation or movement of a head or an upper body of the driver.

5. The image processing device according to claim 1, further comprising
a saliency map generation unit (708) that generates the saliency map according to a state of the driver or a traveling situation of the vehicle, wherein
the processing unit recognizes or discriminates the consciousness level of the driver using the saliency map generated by the saliency map generation unit (708).

6. The image processing device according to claim 5, wherein
the saliency map generation unit (708) generates the saliency map on a basis of information of an obstacle detected around the vehicle;
in particular the image processing device further comprising
a display unit that artificially displays a virtual obstacle on a head up display, wherein
the saliency map generation unit (708) generates the saliency map with further addition of information of the virtual obstacle displayed by the display unit.

7. The image processing device according to claim 1, further comprising
a learning unit that learns personal characteristics of the eye movement of the driver;
in particular wherein
the processing unit recognizes or discriminates the consciousness level of the driver with application of the personal characteristics.

8. The image processing device according to claim 1, wherein
the processing unit determines a dominant eye of the driver and recognizes or discriminates the consciousness level of the driver on a basis of an image of at least the dominant eye included in the region.

9. An image processing method, comprising:
a first image acquisition step of acquiring an image of a driver of a vehicle at a first frame rate;
a region determination step of determining a region including eyes of the driver in the image acquired by the first image acquisition step;
a second image acquisition step of acquiring an image of the region at a second frame rate higher than the first frame rate;
a processing step of processing an image of the region acquired at the second frame rate to recognize or discriminate a consciousness level of the driver; and
a control step of controlling switching of a driving mode of the vehicle according to the consciousness level of the driver recognized or discriminated by the processing step,
wherein, in the processing step, the consciousness level of the driver is recognized or discriminated according to a tracking result of eye movement of the driver on a basis of the image in the region,
wherein, in the processing step, at least one action of saccade and microsaccade of the eyes of the driver is tracked, and
wherein, in the processing step, the consciousness level of the driver is recognized or discriminated on a basis of a comparison result between the eye movement of the driver and a saliency map by detecting the correlation between the direction of the visual line of the driver and the saliency map, wherein the saliency map is a two-dimensional distribution map that represents the probability distribution of positions naturally seen by the driver due to the visual characteristics of a target.

10. A movable body, comprising:
an image pickup unit that is configured to pick up an image of a driver;
a first image acquisition unit (421, 521) that is configured to acquire the picked-up image of the driver at a first frame rate;
a region determination unit (423, 702) that is configured to determine a region including eyes of the driver in the image acquired by the first image acquisition unit;
a second image acquisition unit (424, 524, 703) that is configured to acquire an image of the region at a second frame rate higher than the first frame rate;
a processing unit that is configured to process an image of the region acquired at the second frame rate to recognize or discriminate a consciousness level of the driver; and
a control unit that is configured to control switching of a driving mode according to the consciousness level of the driver recognized or discriminated by the processing unit,
wherein the processing unit is configured to recognize or discriminate the consciousness level of the driver according to a tracking result of eye movement of the driver on a basis of the image in the region,
wherein the processing unit is configured to track at least one action of saccade and microsaccade of the eyes of the driver, and
wherein the processing unit is configured to recognize or discriminate the consciousness level of the driver on a basis of a comparison result between the eye movement of the driver and a saliency map by detecting the correlation between the direction of the visual line of the driver and the saliency map, wherein the saliency map is a two-dimensional distribution map that represents the probability distribution of positions naturally seen by the driver due to the visual characteristics of a target.

11. The movable body according to claim 10, wherein
the image pickup unit is constituted by laminating together three semiconductor substrates including a first semiconductor substrate (510), a second semiconductor substrate (520), and a third semiconductor substrate (530),
the first semiconductor substrate (510) has pixels,
the second semiconductor substrate (530) has a storage unit that stores the image, and
the third semiconductor substrate (520) has at least one of the first image acquisition unit (421, 521), the second image acquisition unit (424, 524, 703), the region determination unit (423, 702), the processing unit, and the control unit.

## Patentansprüche

1. Bildverarbeitungsvorrichtung, die Folgendes aufweist:
eine erste Bilderfassungseinheit (421, 521), die dazu ausgelegt ist, ein Bild eines Fahrers eines Fahrzeugs mit einer ersten Bildrate zu erfassen;
eine Bereichsbestimmungseinheit (423, 702), die dazu ausgelegt ist, einen Bereich zu bestimmen, der die Augen des Fahrers in dem durch die erste Bilderfassungseinheit erfassten Bild enthält;
eine zweite Bilderfassungseinheit (424, 524, 703), die dazu ausgelegt ist, ein Bild des Bereichs mit einer zweiten Bildrate zu erfassen, die höher als die erste Bildrate ist;
eine Verarbeitungseinheit, die dazu ausgelegt ist, ein mit der zweiten Bildrate erfasstes Bild des Bereichs zu verarbeiten, um eine Bewusstseinsstufe des Fahrers zu erkennen oder zu unterscheiden; und
eine Steuereinheit, die dazu ausgelegt ist, das Umschalten eines Fahrmodus des Fahrzeugs gemäß der durch die Verarbeitungseinheit erkannten oder unterschiedenen Bewusstseinsstufe des Fahrers zu steuern,
wobei die Verarbeitungseinheit dazu ausgelegt ist, die Bewusstseinsstufe des Fahrers gemäß einem Verfolgungsergebnis der Augenbewegung des Fahrers auf der Grundlage des Bildes in dem Bereich zu erkennen oder zu unterscheiden,
wobei die Verarbeitungseinheit dazu ausgelegt ist, mindestens eine Aktion von Sakkade und Mikrosakkade der Augen des Fahrers zu verfolgen, und
wobei die Verarbeitungseinheit dazu ausgelegt ist, die Bewusstseinsstufe des Fahrers auf der Grundlage eines Vergleichsergebnisses zwischen der Augenbewegung des Fahrers und einer Salienzkarte zu erkennen oder zu unterscheiden, indem sie die Korrelation zwischen der Richtung der Sichtlinie des Fahrers und der Salienzkarte erfasst, wobei die Salienzkarte eine zweidimensionale Verteilungskarte ist, die die Wahrscheinlichkeitsverteilung von Positionen darstellt, die der Fahrer aufgrund der visuellen Eigenschaften eines Ziels natürlich sieht.

2. Bildverarbeitungsvorrichtung nach Anspruch 1, wobei
die Steuereinheit den Wechsel vom automatisierten Fahren zum manuellen Fahren des Fahrzeugs einschränkt oder untersagt, wenn die Bewusstseinsstufe des Fahrers gleich oder weniger als eine vorgeschriebene Stufe beträgt.

3. Bildverarbeitungsvorrichtung nach Anspruch 1, die ferner Folgendes aufweist:
eine Warneinheit, die ein Warnsignal ausgibt, wenn die Bewusstseinsstufe des Fahrers gleich oder weniger als eine vorgeschriebenen Stufe beträgt.

4. Bildverarbeitungsvorrichtung nach Anspruch 1, wobei
die Bereichsbestimmungseinheit eine Position des Bereichs gemäß der Drehung oder Bewegung eines Kopfes oder eines Oberkörpers des Fahrers korrigiert.

5. Bildverarbeitungsvorrichtung nach Anspruch 1, die ferner Folgendes aufweist:
eine Salienzkartenerzeugungseinheit (708), die die Salienzkarte gemäß einem Zustand des Fahrers oder einer Fahrsituation des Fahrzeugs erzeugt, wobei
die Verarbeitungseinheit die Bewusstseinsstufe des Fahrers anhand der durch die Salienzkartenerzeugungseinheit (708) erzeugten Salienzkarte erkennt oder unterscheidet.

6. Bildverarbeitungsvorrichtung nach Anspruch 5, wobei die Salienzkartenerzeugungseinheit (708) die Salienzkarte auf der Grundlage von Informationen über ein im Umfeld des Fahrzeugs erfasstes Hindernis erzeugt;
wobei die Bildverarbeitungsvorrichtung ferner insbesondere Folgendes aufweist:
eine Anzeigeeinheit, die ein virtuelles Hindernis auf einem Head-up-Display künstlich darstellt, wobei
die Salienzkartenerzeugungseinheit (708) die Salienzkarte unter Hinzufügung weiterer Informationen über das durch die Anzeigeeinheit dargestellte virtuelle Hindernis erzeugt.

7. Bildverarbeitungsvorrichtung nach Anspruch 1, die ferner Folgendes aufweist:
eine Lerneinheit, die die persönlichen Merkmale der Augenbewegungen des Fahrers lernt;
wobei insbesondere
die Verarbeitungseinheit die Bewusstseinsstufe des Fahrers unter Verwendung der persönlichen Merkmale erkennt oder unterscheidet.

8. Bildverarbeitungsvorrichtung nach Anspruch 1, wobei
die Verarbeitungseinheit ein dominantes Auge des Fahrers bestimmt und die Bewusstseinsstufe des Fahrers auf der Grundlage eines Bildes zumindest des dominanten Auges, das in dem Bereich enthalten ist, erkennt oder unterscheidet.

9. Bildverarbeitungsverfahren, das Folgendes aufweist:
einen ersten Bilderfassungsschritt des Erfassens eines Bildes eines Fahrers eines Fahrzeugs mit einer ersten Bildrate;
einen Bereichsbestimmungsschritt des Bestimmens eines Bereichs, der die Augen des Fahrers in dem durch den ersten Bilderfassungsschritt erfassten Bild enthält;
einen zweiten Bilderfassungsschritt des Erfassens eines Bildes des Bereichs mit einer zweiten Bildrate, die höher als die erste Bildrate ist;
einen Verarbeitungsschritt des Verarbeitens eines Bildes des Bereichs, der mit der zweiten Bildrate erfasst wurde, um eine Bewusstseinsstufe des Fahrers zu erkennen oder zu unterscheiden; und
einen Steuerschritt des Steuerns des Umschaltens eines Fahrmodus des Fahrzeugs gemäß der Bewusstseinsstufe des Fahrers, die durch den Verarbeitungsschritt erkannt oder unterschieden wurde,
wobei in dem Verarbeitungsschritt die Bewusstseinsstufe des Fahrers gemäß einem Verfolgungsergebnis der Augenbewegung des Fahrers auf der Grundlage des Bildes in dem Bereich erkannt oder unterschieden wird,
wobei in dem Verarbeitungsschritt mindestens eine Aktion von Sakkade und Mikrosakkade der Augen des Fahrers verfolgt wird, und
wobei in dem Verarbeitungsschritt die Bewusstseinsstufe des Fahrers auf der Grundlage eines Vergleichsergebnisses zwischen der Augenbewegung des Fahrers und einer Salienzkarte erkannt oder unterschieden wird, indem die Korrelation zwischen der Richtung der Sichtlinie des Fahrers und der Salienzkarte erfasst wird, wobei die Salienzkarte eine zweidimensionale Verteilungskarte ist, die die Wahrscheinlichkeitsverteilung von Positionen darstellt, die durch den Fahrer aufgrund der visuellen Eigenschaften eines Ziels natürlich gesehen werden.

10. Bewegbarer Körper, der Folgendes aufweist:
eine Bildaufnahmeeinheit, die dazu ausgelegt ist, ein Bild eines Fahrers aufzunehmen;
eine erste Bilderfassungseinheit (421, 521), die dazu ausgelegt ist, das aufgenommene Bild des Fahrers mit einer ersten Bildrate zu erfassen;
eine Bereichsbestimmungseinheit (423, 702), die dazu ausgelegt ist, einen Bereich zu bestimmen, der die Augen des Fahrers in dem durch die erste Bilderfassungseinheit erfassten Bild enthält;
eine zweite Bilderfassungseinheit (424, 524, 703), die dazu ausgelegt ist, ein Bild des Bereichs mit einer zweiten Bildrate zu erfassen, die höher als die erste Bildrate ist;
eine Verarbeitungseinheit, die dazu ausgelegt ist, ein mit der zweiten Bildrate erfasstes Bild des Bereichs zu verarbeiten, um eine Bewusstseinsstufe des Fahrers zu erkennen oder zu unterscheiden; und
eine Steuereinheit, die dazu ausgelegt ist, das Umschalten eines Fahrmodus gemäß der durch die Verarbeitungseinheit erkannten oder unterschiedenen Bewusstseinsstufe des Fahrers zu steuern,
wobei die Verarbeitungseinheit dazu ausgelegt ist, die Bewusstseinsstufe des Fahrers gemäß einem Verfolgungsergebnis der Augenbewegung des Fahrers auf der Grundlage des Bildes in dem Bereich zu erkennen oder zu unterscheiden,
wobei die Verarbeitungseinheit dazu ausgelegt ist, mindestens eine Aktion von Sakkade und Mikrosakkade der Augen des Fahrers zu verfolgen, und
wobei die Verarbeitungseinheit dazu ausgelegt ist, die Bewusstseinsstufe des Fahrers auf der Grundlage eines Vergleichsergebnisses zwischen der Augenbewegung des Fahrers und einer Salienzkarte zu erkennen oder zu unterscheiden, indem sie die Korrelation zwischen der Richtung der Sichtlinie des Fahrers und der Salienzkarte erfasst, wobei die Salienzkarte eine zweidimensionale Verteilungskarte ist, die die Wahrscheinlichkeitsverteilung von Positionen darstellt, die der Fahrer aufgrund der visuellen Eigenschaften eines Ziels natürlich sieht.

11. Bewegbarer Körper nach Anspruch 10, wobei
die Bildaufnahmeeinheit durch Zusammenlaminieren von drei Halbleitersubstraten einschließlich eines ersten Halbleitersubstrats (510), eines zweiten Halbleitersubstrats (520) und eines dritten Halbleitersubstrats (530) gebildet wird,
das erste Halbleitersubstrat (510) Pixel aufweist,
das zweite Halbleitersubstrat (530) eine Speichereinheit aufweist, die das Bild speichert, und
das dritte Halbleitersubstrat (520) mindestens eine der ersten Bilderfassungseinheit (421, 521), der zweiten Bilderfassungseinheit (424, 524, 703), der Bereichsbestimmungseinheit (423, 702), der Verarbeitungseinheit und der Steuereinheit aufweist.

## Revendications

1. Dispositif de traitement d'images, comprenant :
une première unité d'acquisition d'images (421, 521) qui est configurée pour acquérir une image d'un conducteur d'un véhicule à une première cadence d'images ;
une unité de détermination de zone (423, 702) qui est configurée pour déterminer une zone contenant les yeux du conducteur dans l'image acquise par la première unité d'acquisition d'images ;
une deuxième unité d'acquisition d'images (424, 524, 703) qui est configurée pour acquérir une image de la zone à une deuxième cadence d'images plus élevée que la première cadence d'images ;
une unité de traitement qui est configurée pour traiter une image de la zone acquise à la deuxième cadence d'images afin de reconnaître ou de discerner un niveau de conscience du conducteur ; et
une unité de commande qui est configurée pour commander la commutation d'un mode de conduite du véhicule selon le niveau de conscience du conducteur reconnu ou discerné par l'unité de traitement,
l'unité de traitement étant configurée pour reconnaître ou discerner le niveau de conscience du conducteur selon un résultat de suivi d'un mouvement des yeux du conducteur sur la base de l'image dans la zone,
l'unité de traitement étant configurée pour suivre au moins une action de saccade et de microsaccade des yeux du conducteur, et
l'unité de traitement étant configurée pour reconnaître ou discerner le niveau de conscience du conducteur sur la base d'un résultat de comparaison entre le mouvement des yeux du conducteur et une carte de saillance par détection de la corrélation entre la direction de la ligne de visée du conducteur et la carte de saillance, la carte de saillance étant une carte de distribution bidimensionnelle qui représente la distribution des probabilités de positions naturellement perçues par le conducteur du fait des caractéristiques visuelles d'une cible.

2. Dispositif de traitement d'images selon la revendication 1, dans lequel
l'unité de commande limite ou bloque la commutation d'une conduite automatique sur une conduit manuelle du véhicule lorsque le niveau de conscience du conducteur est inférieur ou égal à un niveau prescrit.

3. Dispositif de traitement d'images selon la revendication 1, comprenant en outre
une unité d'alerte qui émet une alerte lorsque le niveau de conscience du conducteur est inférieur ou égal à un niveau prescrit.

4. Dispositif de traitement d'images selon la revendication 1, dans lequel
l'unité de détermination de zone corrige une position de la zone selon une rotation ou un mouvement de la tête ou du haut du corps du conducteur.

5. Dispositif de traitement d'images selon la revendication 1, comprenant en outre
une unité de génération de carte de saillance (708) qui génère la carte de saillance selon un état du conducteur ou une situation de circulation du véhicule,
l'unité de traitement reconnaissant ou discernant le niveau de conscience du conducteur à l'aide de la carte de saillance générée par l'unité de génération de carte de saillance (708).

6. Dispositif de traitement d'images selon la revendication 5, dans lequel
l'unité de génération de carte de saillance (708) génère la carte de saillance sur la base d'informations relatives à un obstacle détecté autour du véhicule ;
plus particulièrement, le dispositif de traitement d'images comprenant en outre
une unité d'affichage qui affiche de manière artificielle un obstacle virtuel sur un affichage tête haute,
l'unité de génération de carte de saillance (708) générant la carte de saillance avec l'ajout en outre d'informations relatives à l'obstacle virtuel affiché par l'unité d'affichage.

7. Dispositif de traitement d'images selon la revendication 1, comprenant en outre
une unité d'apprentissage qui apprend des caractéristiques personnelles du mouvement des yeux du conducteur ;
plus particulièrement
l'unité de traitement reconnaissant ou discernant le niveau de conscience du conducteur en appliquant les caractéristiques personnelles.

8. Dispositif de traitement d'images selon la revendication 1, dans lequel
l'unité de traitement détermine un œil dominant du conducteur et reconnaît ou discerne le niveau de conscience du conducteur sur la base d'une image d'au moins l'œil dominant contenue dans la zone.

9. Procédé de traitement d'images, comprenant :
une première étape d'acquisition d'images consistant à acquérir une image d'un conducteur d'un véhicule à une première cadence d'images ;
une étape de détermination de zone consistant à déterminer une zone contenant les yeux du conducteur dans l'image acquise à la première unité d'acquisition d'images ;
une deuxième étape d'acquisition d'images consistant à acquérir une image de la zone à une deuxième cadence d'images plus élevée que la première cadence d'images ;
une étape de traitement consistant à traiter une image de la zone acquise à la deuxième cadence d'images afin de reconnaître ou de discerner un niveau de conscience du conducteur ; et
une étape de commande consistant à commander la commutation d'un mode de conduite du véhicule selon le niveau de conscience du conducteur reconnu ou discerné à l'étape de traitement,
à l'étape de traitement, le niveau de conscience du conducteur étant reconnu ou discerné selon un résultat de suivi d'un mouvement des yeux du conducteur sur la base de l'image dans la zone,
à l'étape de traitement, au moins une action de saccade et de microsaccade des yeux du conducteur étant suivie, et
à l'étape de traitement, le niveau de conscience du conducteur étant reconnu ou discerné sur la base d'un résultat de comparaison entre le mouvement des yeux du conducteur et une carte de saillance par détection de la corrélation entre la direction de la ligne de visée du conducteur et la carte de saillance, la carte de saillance étant une carte de distribution bidimensionnelle qui représente la distribution des probabilités de positions naturellement perçues par le conducteur du fait des caractéristiques visuelles d'une cible.

10. Corps en mouvement, comprenant :
une unité de capture d'images qui est configurée pour capturer une image d'un conducteur ;
une première unité d'acquisition d'images (421, 521) qui est configurée pour acquérir l'image capturée du conducteur à une première cadence d'images ;
une unité de détermination de zone (423, 702) qui est configurée pour déterminer une zone contenant les yeux du conducteur dans l'image acquise par la première unité d'acquisition d'images ;
une deuxième unité d'acquisition d'images (424, 524, 703) qui est configurée pour acquérir une image de la zone à une deuxième cadence d'images plus élevée que la première cadence d'images ;
une unité de traitement qui est configurée pour traiter une image de la zone acquise à la deuxième cadence d'images afin de reconnaître ou de discerner un niveau de conscience du conducteur ;
une unité de commande qui est configurée pour commander la commutation d'un mode de conduite selon le niveau de conscience du conducteur reconnu ou discerné par l'unité de traitement,
l'unité de traitement étant configurée pour reconnaître ou discerner le niveau de conscience du conducteur selon un résultat de suivi d'un mouvement des yeux du conducteur sur la base de l'image dans la zone,
l'unité de traitement étant configurée pour suivre au moins une action de saccade et de microsaccade des yeux du conducteur, et
l'unité de traitement étant configurée pour reconnaître ou discerner le niveau de conscience du conducteur sur la base d'un résultat de comparaison entre le mouvement des yeux du conducteur et une carte de saillance par détection de la corrélation entre la direction de la ligne de visée du conducteur et la carte de saillance, la carte de saillance étant une carte de distribution bidimensionnelle qui représente la distribution des probabilités de positions naturellement perçues par le conducteur du fait des caractéristiques visuelles d'une cible.

11. Corps en mouvement selon la revendication 10, dans lequel
l'unité de capture d'images est formée en laminant ensemble trois substrats semi-conducteurs comportant un premier substrat semi-conducteur (510), un deuxième substrat semi-conducteur (520) et un troisième substrat semi-conducteur (530),
le premier substrat semi-conducteur (510) étant pourvu de pixels,
le deuxième substrat semi-conducteur (530) étant pourvu d'une unité de stockage qui stocke l'image, et
le troisième substrat semi-conducteur (520) étant pourvu d'au moins une unité parmi la première unité d'acquisition d'images (421, 521), la deuxième unité d'acquisition d'images (424, 524, 703), l'unité de détermination de zone (423, 702), l'unité de traitement et l'unité de commande.
